# EUROPEAN PATENT APPLICATION

(11) **EP 4 317 178 A1**
(43) Date of publication of application: **07.02.2024**
(21) Application number: 22810673.8
(22) Date of filing: 30.05.2022
(51) Int. Cl.: C07K 14/575, C07K 14/605, A61K 38/22, A61K 38/26, A61P 1/16

(54) **PREPARATION AND APPLICATION OF POLYPEPTIDE**

(30) Priority: 28.05.2021 CN 202110594662; 19.07.2021 CN 202110814116; 15.02.2022 CN 202210138835; 18.05.2022 CN 202210552077
(71) Applicant: Guangdong Raynovent Biotech Co., Ltd., Huangpu District Guangzhou 510700 (CN)
(72) Inventor: JIANG, Zhigan, Shanghai 200131 (CN); CHEN, Xiaoxin, Guangzhou, Guangdong 510700 (CN); PAN, Zhixiang, Shanghai 200131 (CN); CHEN, Minglu, Guangzhou, Guangdong 510700 (CN); HE, Haiying, Shanghai 200131 (CN); HUANG, Jianzhou, Guangzhou, Guangdong 510700 (CN); HU, Guoping, Shanghai 200131 (CN); LIU, Zhuowei, Guangzhou, Guangdong 510700 (CN); LI, Jian, Shanghai 200131 (CN); LONG, Chaofeng, Guangzhou, Guangdong 510700 (CN); CHEN, Shuhui, Shanghai 200131 (CN)
(74) Representative: Potter Clarkson
(86) International application number: PCT/CN2022/095859
(87) International publication number: WO 2022/247950

(57) **Abstract**

Preparation and application of a series of polypeptides, and specifically disclosed is a polypeptide having a sequence as shown in formula (II).

YAibEGT FTSDY SIAibLD KIAQK AFVKW LIAGG PSSGA PPPS₀ (II).

## Description

This application claims priorities to Chinese Patent Application No. 2021105946626 filed on May 28, 2021, Chinese Patent Application No. 2021108141169 filed on July 19, 2021, Chinese Patent Application No. 2022101388358 filed on February 15, 2022, and Chinese Patent Application No. 2022105520774 filed on May. 18, 2022, the entire contents of which are incorporated herein by reference.

### TECHNICAL FIELD

The present application relates to preparation and application of a series of polypeptides, and specifically to a polypeptide having a sequence as shown in formula (II).

### BACKGROUND

The global incidence of non-alcoholic fatty liver disease (NAFLD) is as high as 25%, with the global incidence of non-alcoholic steatohepatitis (NASH) accounting for approximately 3% to 8%. Some patients with NASH will further progress to cirrhosis and liver cancer, which is currently one of the leading causes of end-stage liver disease and liver transplantation. The pathogenesis of NASH is complex, and there is no FDA-approved drug for the treatment of this disease at present. Several preclinical studies have found that glucose-dependent insulinotropic polypeptide (GIP)/glucagon-like peptide-1 (GLP-1) dual agonists can be used for the treatment of NASH. Clinical studies of Tirzepatide, a GLP-1/GIP dual agonist under the investigation by Eli Lilly and Company, have demonstrated that it can improve NASH-related transaminase and other relevant markers, showing its potential as a treatment for NASH.

GLP-1 agonists can be used to treat NASH synergistically through multiple pathways. For example, GLP-1 agonists can reduce the circulating levels of tumor necrosis factor (TNF)-α, interleukin IL-1β, IL-6, CD163, and hsCRP, thus achieving an anti-inflammatory effect. GIP, a polypeptide secreted by neuroendocrine K cells in the small intestine, can further alleviate hepatic lipid synthesis on the basis of GLP-1 agonist treatment. Therefore, GLP-1/GIP dual agonists have a synergistic effect on the treatment of NASH.

### SUMMARY OF THE INVENTION

The present application provides a polypeptide having a sequence as shown in formula (II),

YAibEGT FTSDY SIAibLD KIAQK AFVKW LIAGG PSSGA PPPS₀ **(II)**

which has modifications as below:
1) the amino groups on the side chains of lysine residues at positions i and i+3 or the amino groups on the side chains of lysine residues at positions j and j+4 are attached to 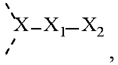 wherein i is 17 (i.e., when i is 17, isoleucine residue at position 17 is replaced by lysine residue first, and the amino group on the side chain of the replaced lysine residue is then attached to 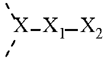 together with the amino group on the side chain of lysine residue at position 20), or wherein j is 20; and
2) additional 0 to 2 amino acids on the polypeptide of the sequence as shown in formula (II) are replaced;
   wherein,
   Aib has a structure of 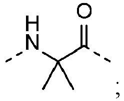
   S₀ is selected from the group consisting of 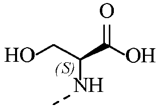 and 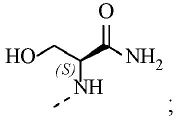
   X is selected from the group consisting of 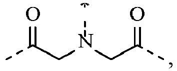 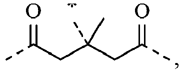 and 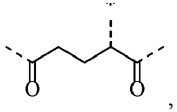 wherein, "*" indicates the position attached to Xi;
   X₁ is selected from the group consisting of a single bond, -C(=O)-, -O-C(=O)-, and -N(Ri)-C(=O)-;
   Ri is selected from the group consisting of H and C₁₋₃ alkyl;
   X₂ is selected from the group consisting of 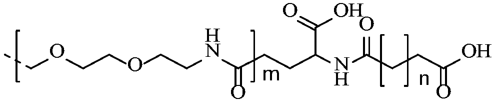 and 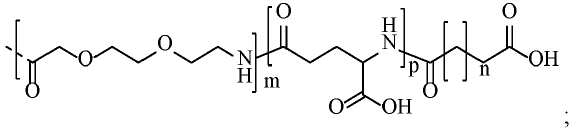
   m is selected from 2, 3, and 4;
   n is selected from 15, 16, 17, 18, and 19;
   p is selected from 1 and 2.

The present application provides a polypeptide having a sequence as shown in formula (P),

YAibEGT FTSDY SIAibLD KKAQK AFVKW LIAGG PSSGA PPPS₀ **(P)**

which has modifications as below:
1) the amino groups on the side chains of lysine residues at positions 17 and 20 are attached to 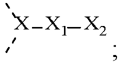 and
2) 0 to 2 amino acids on the polypeptide of the sequence as shown in formula (P) are replaced; wherein,
   Aib has a structure of 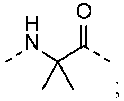
   S₀ is selected from the group consisting of 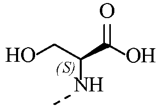 and 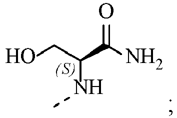
   X is selected from the group consisting of 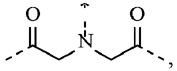 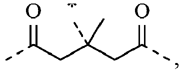 and 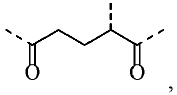 wherein, "*" indicates the position attached to Xi;
   X₁ is selected from the group consisting of a single bond, -C(=O)-, -O-C(=O)-, and -N(Ri)-C(=O)-;
   Ri is selected from the group consisting of H and C₁₋₃ alkyl;
   X₂ is selected from the group consisting of 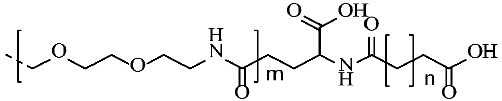 and 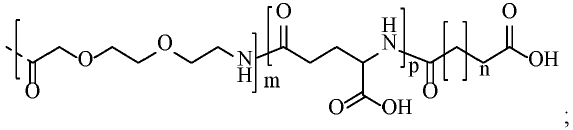
   m is selected from 2, 3, and 4;
   n is selected from 15, 16, 17, 18, and 19;
   p is selected from 1 and 2.

The present application provides a polypeptide having a sequence as shown in formula (II),

YAibEGT FTSDY SIAibLD KIAQK AFVKW LIAGG PSSGA PPPS₀ **(II)**

which has modifications as below:
1) the amino groups on the side chains of lysine residues at positions i and i+3 or the amino groups on the side chains of lysine residues at positions j and j+4 are attached to 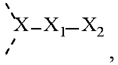 wherein i is 17 (i.e., when i is 17, isoleucine residue at position 17 is replaced by lysine residue first, and the amino group on the side chain of the replaced lysine residue is then attached to 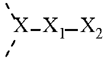 together with the amino group on the side chain of lysine residue at position 20), or wherein j is 20, and
2) additional 0 to 2 amino acids on the polypeptide of the sequence as shown in formula (II) are replaced;
   wherein,
   Aib has a structure of 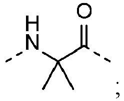
   S₀ is selected from the group consisting of 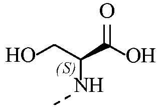 and 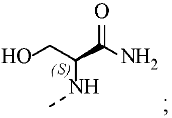
   X is selected from the group consisting of 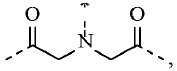 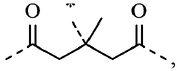 and 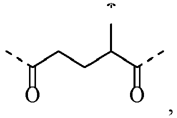 wherein, "*" indicates the position attached to Xi;
   X₁ is selected from the group consisting of a single bond, -C(=O)-, -O-C(=O)-, and -N(Ri)-C(=O)-;
   Ri is selected from the group consisting of H and C₁₋₃ alkyl;
   X₂ is selected from the group consisting of 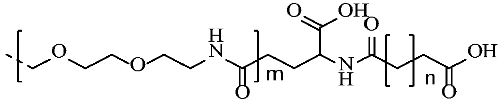 and 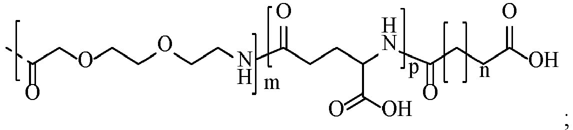
   m is selected from 2, 3, and 4;
   n is selected from 15, 16, 17, 18, and 19;
   p is selected from 1 and 2.

In some embodiments of the present application, 0 to 2 amino acids at position 21, 23, or 24 of the polypeptide are replaced, with other variables being defined herein.

In some embodiments of the present application, the polypeptide has a sequence selected from the group consisting of sequences as shown in formulas (II-1), (II-2), (II-3), (II-4), (III-6), (IV-7), (IV-8), (IV-9), and (IV-10),

YAibEGT FTSDY SIAibLD KIAQK AFVKW LIAGG PSSGAPPPS-NH₂ (**II-1**)

YAibEGT FTSDY SIAibLD KIAQK EFVKW LIAGG PSSGA PPPS-NH₂ **(II-2)**

YAibEGT FTSDY SIAibLD KIAQK AFIKW LIAGG PSSGA PPPS-NH₂ **(II-3)**

YAibEGT FTSDY SIAibLD KIAQK AFVKW LLAGG PSSGA PPPS-NH₂ **(II-4)**

YAibEGT FTSDY SIAibLD KKAQK AFVEW LIAGG PSSGA PPPS-NH₂ **(III-6)**

YAibEGT FTSDY SIAibLD KKAQK AFVQW LIAGG PSSGA PPPS-NH₂ **(IV-7)**

YAibEGT FTSDY SIAibLD KKAQK AFVAW LIAGG PSSGA PPPS-NH₂ **(IV-8)**

YAibEGT FTSDY SIAibLD KKAQK AFVIW LIAGG PSSGA PPPS-NH₂ **(IV-9)**

YAibEGT FTSDY SIAibLD KKAQK EFVEW LIAGG PSSGA PPPS-NH₂ **(IV-10)**

which have modifications as below: the amino groups on the side chains of lysine residues at positions 17 and 20 or the amino groups on the side chains of lysine residues at positions 20 and 24 are attached to 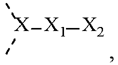 wherein,
Aib, X, Xi, and X₂ are as defined herein.

In some embodiments of the present application, the above Ri is selected from H, with other variables being defined herein.

In some embodiments of the present application, the above X₁ is selected from a single bond, - C(=O)-, -O-C(=O)-, and -NH-C(=O)-, with other variables being defined herein.

In some embodiments of the present application, the above m is selected from 2, with other variables being defined herein.

In some embodiments of the present application, the above n is selected from 15 and 17, with other variables being defined herein.

In some embodiments of the present application, the above X₂ is selected from the group consisting of 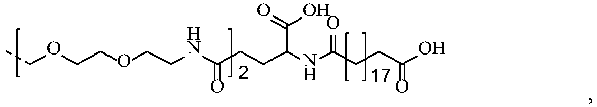 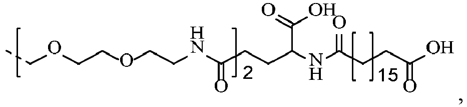 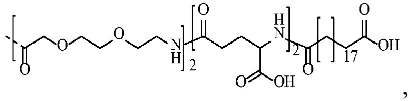 and 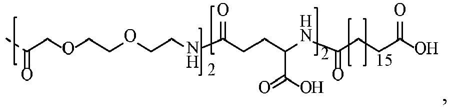 with other variables being defined herein.

In some embodiments of the present application, the above X₂ is selected from the group consisting of 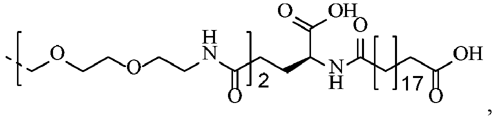 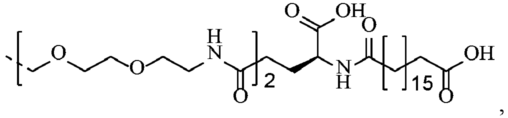 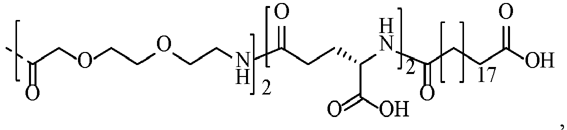 and 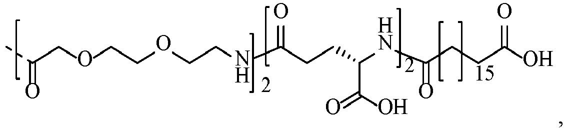 with other variables being defined herein.

In some embodiments of the present application, the amino groups on the side chains of lysine residues at positions i and i+3 or the amino groups on the side chains of lysine residues at positions j and j+4 are attached to 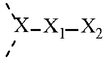 to form 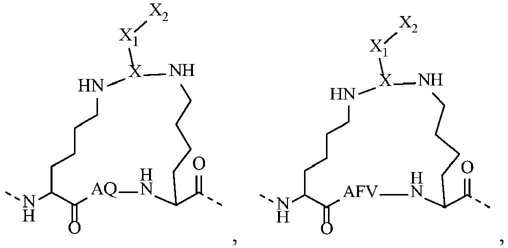 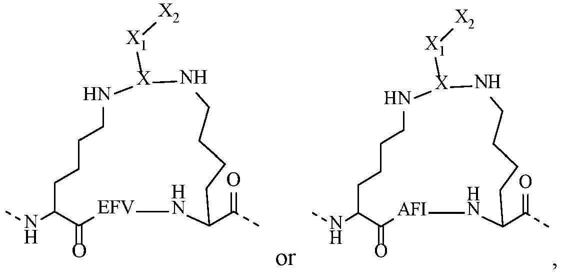 with other variables being defined herein.

In some embodiments of the present application, the amino groups on the side chains of lysine residues at positions i and i+3 or the amino groups on the side chains of lysine residues at positions j and j+4 are attached to 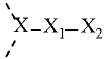 to form 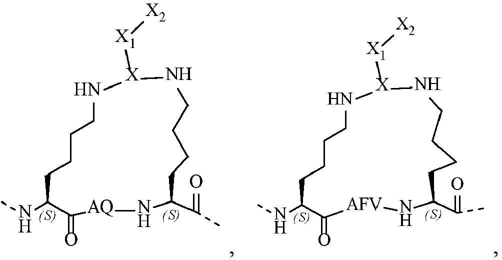 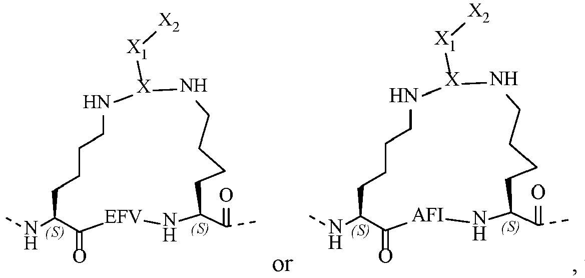 with other variables being defined herein.

In some embodiments of the present application, the above 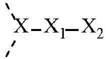 is selected from the group consisting of 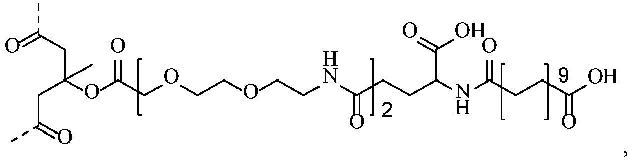 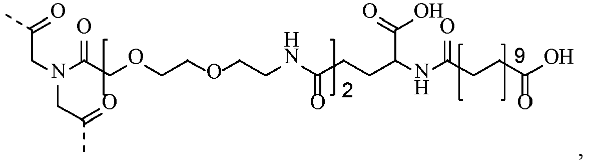 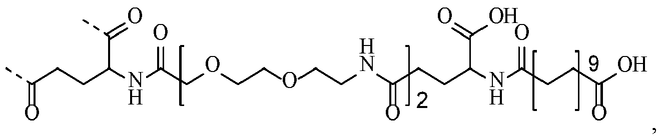 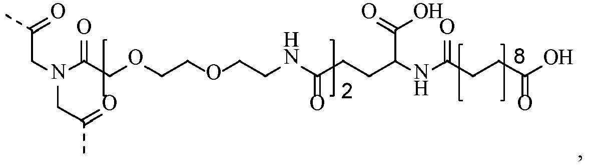 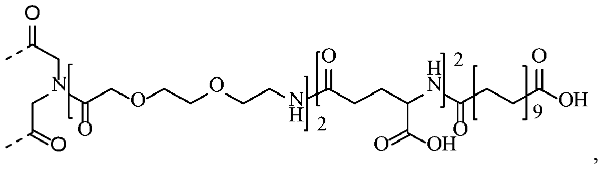 and 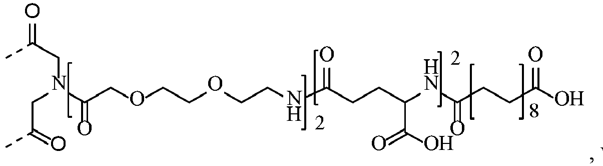 with other variables being defined herein.

In some embodiments of the present application, the above group consisting of 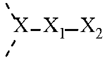 is selected from the 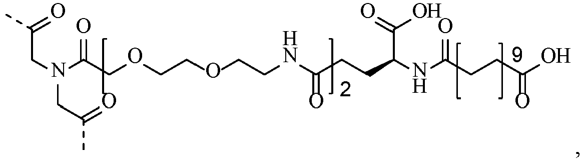 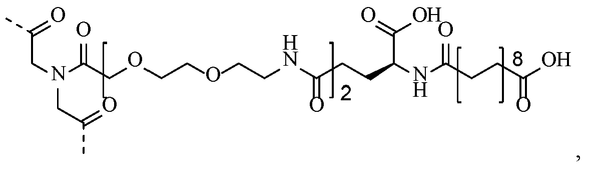 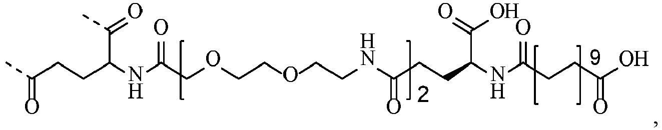 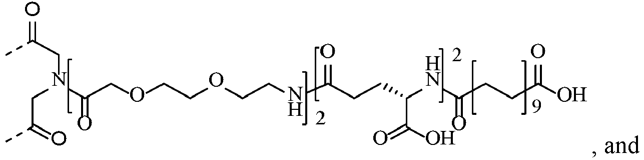 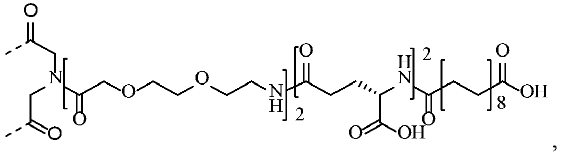 with other variables being defined herein.

The present application provides a polypeptide having a sequence as shown in formula (II),

YAibEGT FTSDY SIAibLD KIAQK AFVKW LIAGG PSSGA PPPS₀ **(II)**

which has modifications as below:
1) the amino groups on the side chains of lysine residues at positions i and i+3 or the amino groups on the side chains of lysine residues at positions j and j+4 are attached to 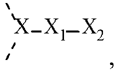 wherein i is 17, or wherein j is 20;
2) 0 to 2 amino acids on the polypeptide as shown in formula (II) are replaced;
   wherein,
   Aib has a structure of 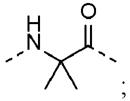
   S₀ is selected from the group consisting of 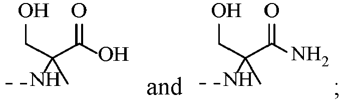
   X is selected from the group consisting of 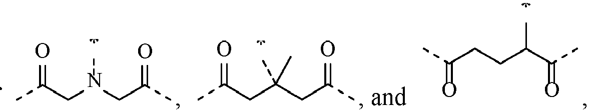 wherein, "*" indicates the position attached to Xi;
   X₁ is selected from the group consisting of -C(=O)-, -O-C(=O)-, and -N(Ri)-C(=O)-;
   Ri is selected from the group consisting of H and C₁₋₃ alkyl;
   X₂ is selected from 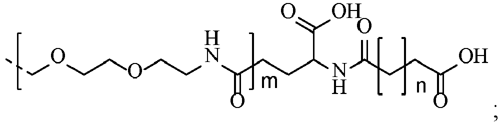
   m is selected from 2, 3, and 4;
   n is selected from 15, 16, 17, 18, and 19.

The present application provides a polypeptide having a sequence as shown in formula (II),

YAibEGT FTSDY SIAibLD KIAQK AFVKW LIAGG PSSGA PPPS₀ **(II)**

which has modifications as below:
1) the amino groups on the side chains of lysine residues at positions i and i+3 or the amino groups on the side chains of lysine residues at positions j and j+4 are attached to 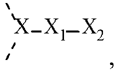 wherein i is 17, or wherein j is 20;
2) 0 to 1 amino acid on the polypeptide as shown in formula (II) are replaced; wherein,
   Aib has a structure of 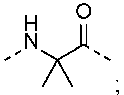
   S₀ is selected from the group consisting of 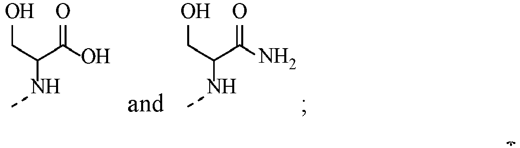
   X is selected from the group consisting of 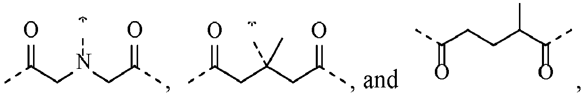 wherein, "*" indicates the position attached to Xi;
   X₁ is selected from the group consisting of -C(=O)-, -O-C(=O)-, and -N(Ri)-C(=O)-;
   Ri is selected from the group consisting of H and C₁₋₃ alkyl;
   X₂ is selected from 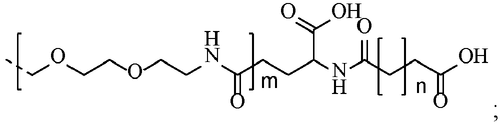
   m is selected from 2, 3, and 4;
   n is selected from 15, 16, 17, 18, and 19.

In some embodiments of the present application, the polypeptide has a sequence selected from the group consisting of sequences as shown in formulas (II-1), (II-2), (II-3), (II-4), (II-5), and (III-6),

YAibEGT FTSDY SIAibLD KIAQK AFVKW LIAGG PSSGA PPPS-NH₂ **(II-1)**

YAibEGT FTSDY SIAibLD KIAQK EFVKW LIAGG PSSGA PPPS-NH₂ **(II-2)**

YAibEGT FTSDY SIAibLD KIAQK AFIKW LIAGG PSSGA PPPS-NH₂ **(II-3)**

YAibEGT FTSDY SIAibLD KIAQK AFVKW LLAGG PSSGA PPPS-NH₂ **(II-4)**

YAibEGT FTSDY SIAibLD KKAQK AFVKW LIAGG PSSGA PPPS-NH₂ **(II-5)**

YAibEGT FTSDY SIAibLD KKAQK AFVEW LIAGG PSSGA PPPS-NH₂ **(III-6)**

which have modifications as below:
the amino groups on the side chains of lysine residues at positions 17 and 20 or the amino groups on the side chains of lysine residues at positions 20 and 24 are attached to 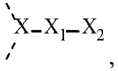 wherein,
Aib, X, X₁, and X₂ are as defined herein.

In some embodiments of the present application, the above Ri is selected from H, with other variables being defined herein.

In some embodiments of the present application, the above X₁ is selected from -C(=O)-, -O-C(=O)-, and -NH-C(=O)-, with other variables being defined herein.

In some embodiments of the present application, the above m is selected from 2, with other variables being defined herein.

In some embodiments of the present application, the above n is selected from 17, with other variables being defined herein.

In some embodiments of the present application, the above X₂ is selected from 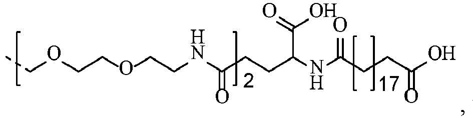 with other variables being defined herein.

In some embodiments of the present application, the amino groups on the side chains of lysine residues at positions i and i+3 or the amino groups on the side chains of lysine residues at positions j and j+4 are attached to 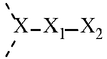 to form 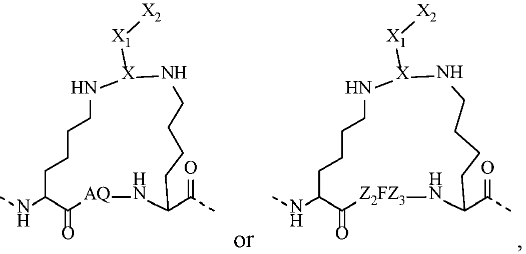 wherein Z₂FZ₃ is AFV, EFV or AFI, with other variables being defined herein.

In some embodiments of the present application, the amino groups on the side chains of lysine residues at positions i and i+3 or the amino groups on the side chains of lysine residues at positions j and j+4 are attached to 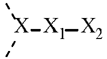 to form 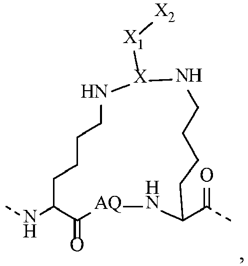 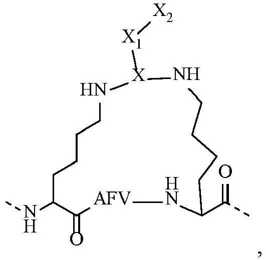 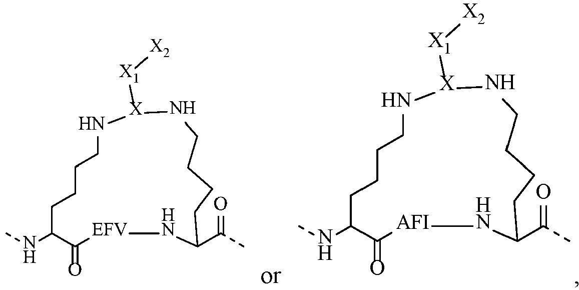 with other variables being defined herein.

In some embodiments of the present application, the above 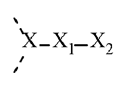 is selected from the group consisting of 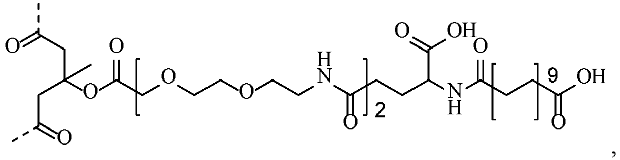 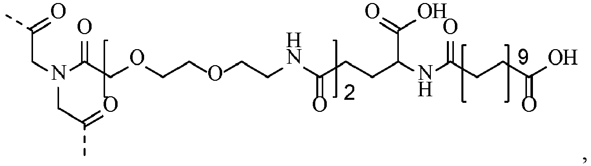 and 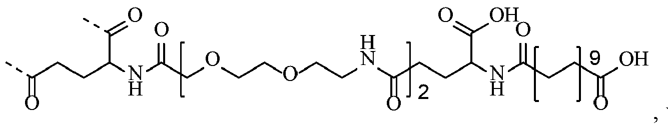 with other variables being defined herein.

The present application also encompasses some embodiments formed by any combination of the above variables.

The present application further provides a polypeptide selected from the group consisting of the formulas below, 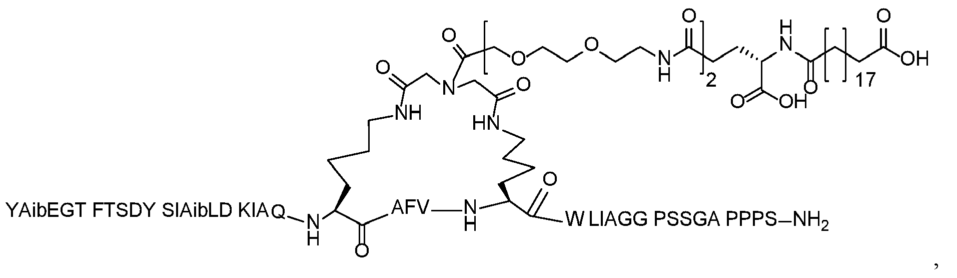 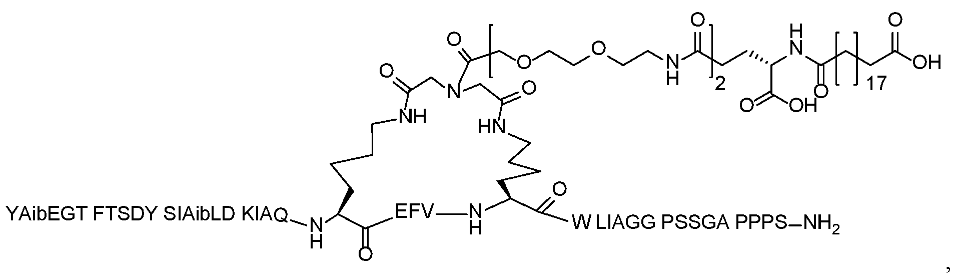 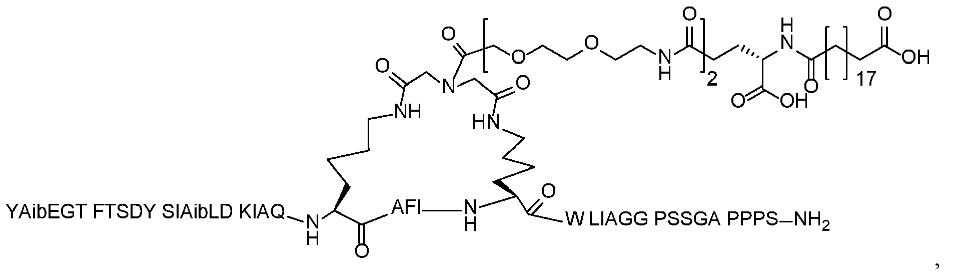 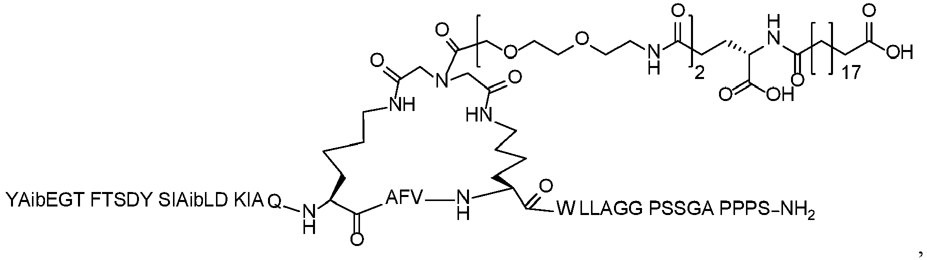 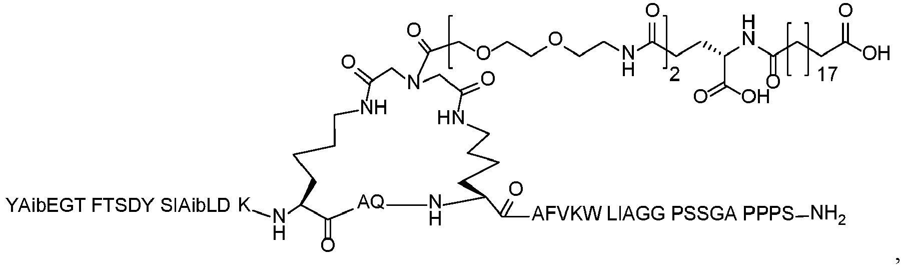 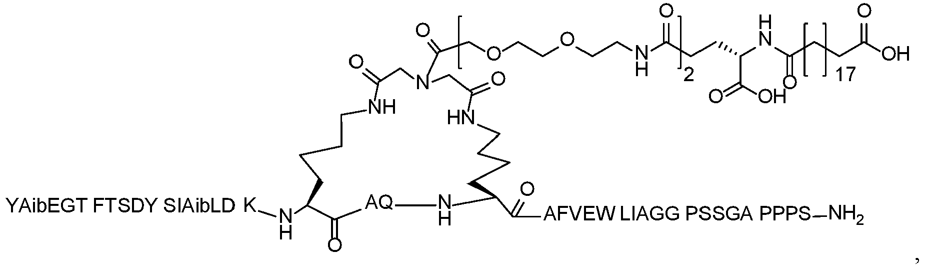 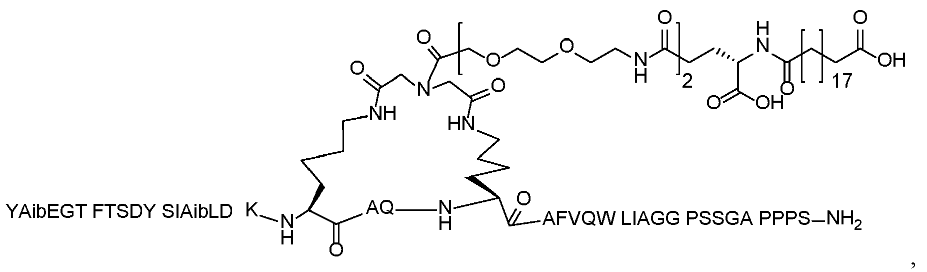 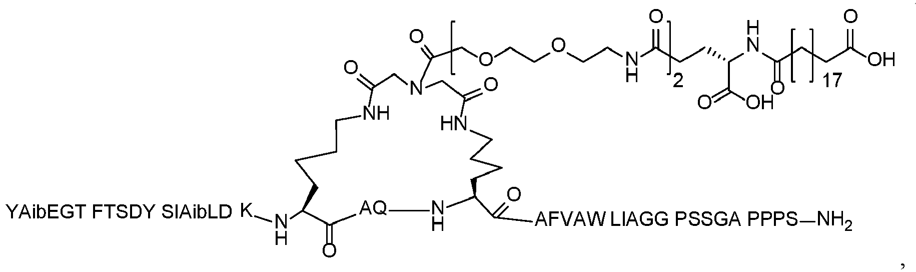 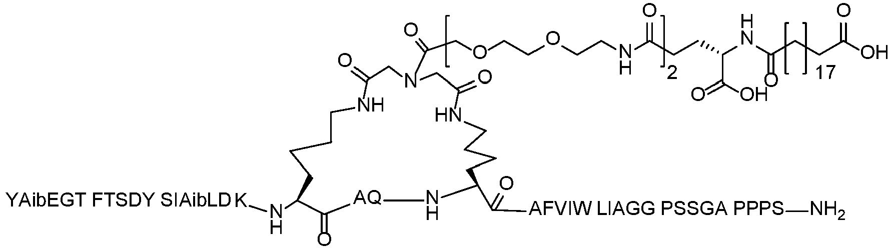 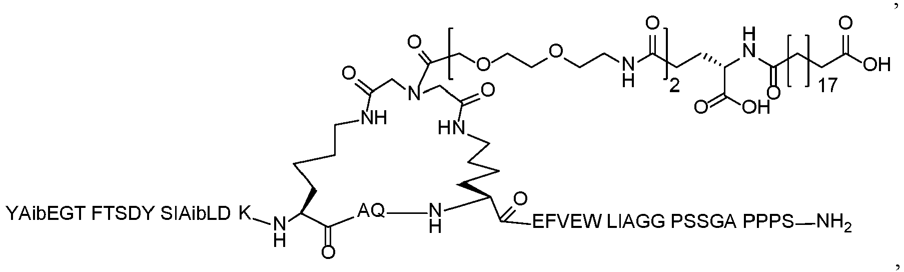 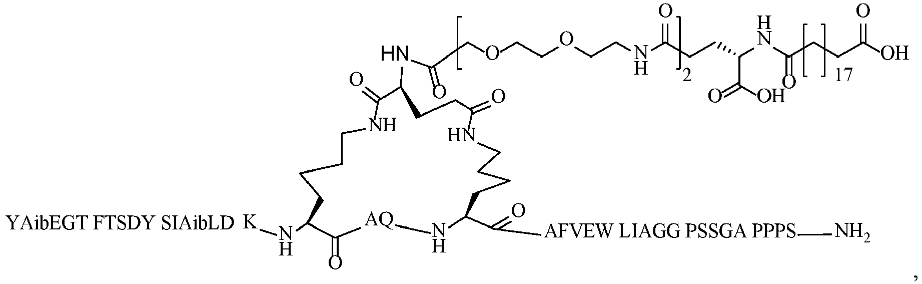 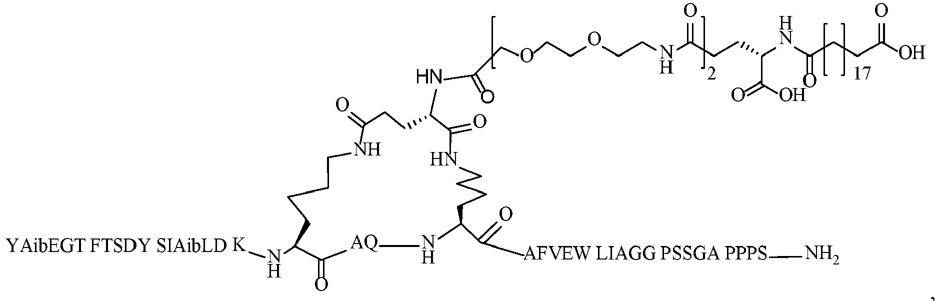 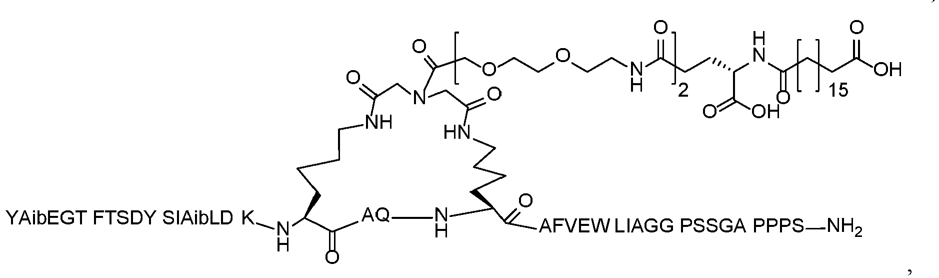 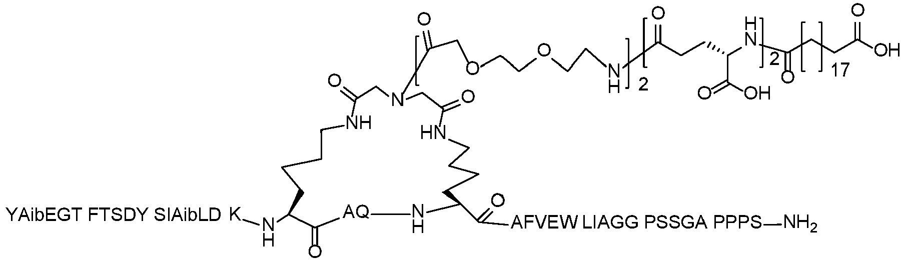 and

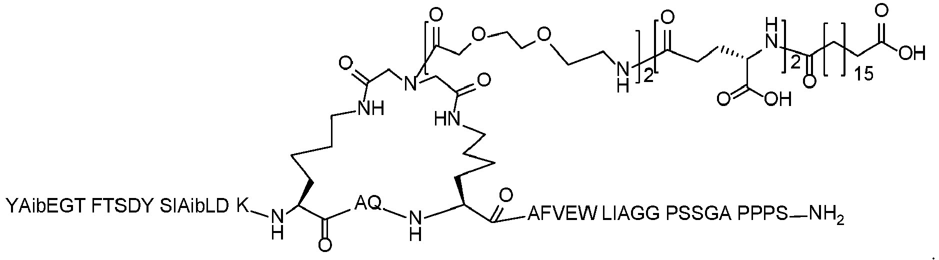

The present application further provides a pharmaceutical composition, which includes a therapeutically effective amount of the above polypeptide compound or a pharmaceutically acceptable salt thereof, as an active ingredient, and a pharmaceutically acceptable carrier.

In some embodiments of the present application, provided is an application of the above polypeptide compound or a pharmaceutically acceptable salt thereof or the above pharmaceutical composition in the preparation of drugs for treating non-alcoholic fatty liver disease (NAFLD) and non-alcoholic steatohepatitis (NASH).

In some embodiments of the present application, the above polypeptide compound or a pharmaceutically acceptable salt thereof or the above pharmaceutical composition is administered once every 3 days, once every 4 days, once a week, or once every two weeks.

### Technical effect

The polypeptide of the present application exhibits very strong agonistic activity on GLP-1R/GIPR; the polypeptide compound of the present application exhibits excellent pharmacokinetic property, plasma stability and extremely high plasma protein binding; and the compound of the present application can significantly improve NAS score in STZ-NASH mouse model.

### Definition and Illustration

Unless otherwise indicated, the following terms and phrases used herein are intended to have the following meanings. A particular term or phrase should not be considered indefinite or unclear in the absence of a particular definition but should be understood in its common meaning. When a trade name is described herein, it is intended to refer to its corresponding commodity or its active ingredient.

The term "pharmaceutically acceptable" as used herein refers to the compounds, materials, compositions and/or dosage forms which are suitable for use in contact with human and animal tissues under the reliable medical judgment, without undue toxicity, irritation, allergic response or other problems or complications, and commensurate with a reasonable benefit/risk ratio.

The term "pharmaceutically acceptable salt" refers to a salt of the compound of the present application, which is prepared from a compound having a specific substituent found in the present application and a relatively non-toxic acid or base. When the compound of the present application contains a relatively acidic functional group, a base addition salt can be obtained by contacting such compound with a sufficient amount of base in a pure solution or a suitable inert solvent. Pharmaceutically acceptable base addition salts include sodium, potassium, calcium, ammonium, organic amine, or magnesium salts or similar salts. When the compound of the present application contain a relatively basic functional group, an acid addition salt can be obtained by contacting such compound with a sufficient amount of acid in a pure solution or a suitable inert solvent. Examples of pharmaceutically acceptable acid addition salts include salts of inorganic acids including, such as, hydrochloric acid, hydrobromic acid, nitric acid, carbonic acid, bicarbonate, phosphoric acid, monohydrogen phosphate, dihydrogen phosphate, sulfuric acid, hydrosulfate, hydroiodic acid, phosphorous acid, etc.; and salts of organic acids including, such as, acetic acid, propionic acid, isobutyric acid, maleic acid, malonic acid, benzoic acid, succinic acid, octanedioic acid, fumaric acid, lactic acid, mandelic acid, phthalic acid, benzenesulfonic acid, p-toluenesulfonic acid, citric acid, tartaric acid, and methanesulfonic acid or other similar acids; and salts of amino acids (e.g., arginine, etc.), and salts of organic acids such as glucuronic acid or the like. Some specific compounds of the present application contain both basic and acidic functional groups and can thus be converted to either base or acid addition salts.

The pharmaceutically acceptable salts of the present application can be synthesized from parent compounds containing acid or base group through conventional chemical methods. Generally, such salts are prepared by reacting such compounds in form of free acid or base with a stoichiometric amount of an appropriate base or acid in water or an organic solvent or a mixture of both.

"Amino acids" refer to naturally occurring or synthesized amino acids, as well as amino acid analogues and amino acid mimetics that function similarly to naturally occurring amino acids. Naturally occurring amino acids are those encoded by genetic codes, as well as those modified amino acids, such as hydroxyproline, γ-carboxyglutamic acid, and O-phosphoserine. Amino acid analogues refer to compounds having the same basic chemical structures (e.g., α-carbon bound to hydrogen, carboxyl group, amino group, and R group) as those of naturally occurring amino acids, such as homoserine, norleucine, methionine sulfoxide, and methionine methylsulfonium. Such analogues may have modified R groups (e.g., norleucine) or modified peptide backbone, but retain the same basic chemical structures as those of naturally occurring amino acids. Amino acid mimetics refer to chemical compounds that have different chemical structures from those of conventional amino acids but perform a similar function to naturally occurring amino acids.

As used herein, A or Ala represents alanine with a structure of 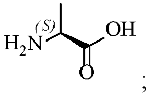 R or Arg represents arginine with a structure of 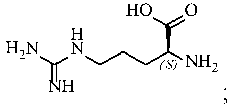 N or Asn represents asparagine with a structure of 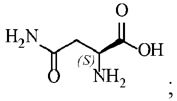 D or Asp represents aspartic acid with a structure of 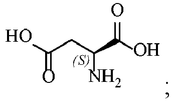 C or Cys represents cysteine with a structure of 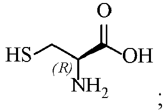 Q or Gln represents glutamine with a structure of 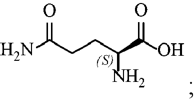 E or Glu represents glutamic acid with a structure of 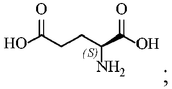 G or Gly represents glycine with a structure of 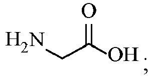 H or His represents histidine with a structure of 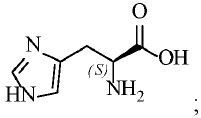 I or Ile represents isoleucine with a structure of 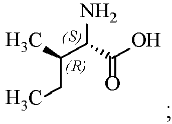 L or Leu represents leucine with a structure of 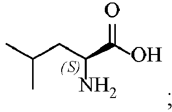 K or Lys represents lysine with a structure of 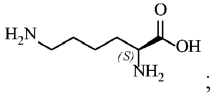 M or Met represents methionine with a structure of 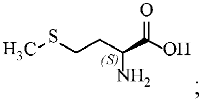 F or Phe represents phenylalanine with a structure of 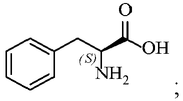 P or Pro represents proline with a structure of 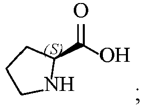 S or Ser represents serine with a structure of 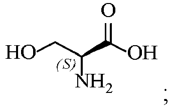 T or Thr represents threonine with a structure of 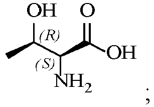 W or Trp represents tryptophan with a structure of 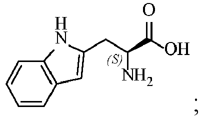 Y or Tyr represents tyrosine with a structure of 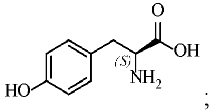 V or Val represents valine with a structure of 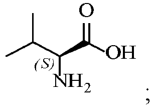 and Fmoc-AEEA-OH represents

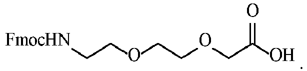

The term "treatment" includes inhibiting, alleviating, stopping or reversing the progression or severity of an existing symptom or disease.

Unless otherwise indicated, the term "isomer" is intended to include geometrical isomers, cis-trans isomers, stereoisomers, enantiomers, optical isomers, diastereomers, and tautomers.

The compound of the present application may exist in specific geometric or stereoisomeric forms. The present application contemplates all such compounds, including cis and trans isomers, (-)- and (+)- enantiomers, (*R*)- and (*S*)-enantiomers, diastereomers, (*D*)-isomers, (*L*)-isomers, and their racemic mixtures and other mixtures, such as enantiomerically or diastereomerically enriched mixtures, all of which are subject to the scope of the present application. Additional asymmetric carbon atoms may be present in substituents such as alkyl groups. All these isomers and mixtures thereof are all included within the scope of the present application.

Unless otherwise indicated, the term "enantiomers" or "optical isomers" refers to stereoisomers that are mirror images of each other.

Unless otherwise indicated, the term "cis-trans isomers" or "geometrical isomers" refers to isomers that result from the restricted rotation around double bonds or single bonds of cyclic carbon atoms.

Unless otherwise indicated, the term "diastereomers" refers to stereoisomers that have two or more chiral centers and exhibit non-mirror-image relationships between molecules.

Unless otherwise indicated, "(+)" represents dextro, "(-)" represents levo, "(±)" represents racemic.

Unless otherwise indicated, a wedge solid line bond ( 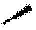 ) and a wedge dotted line bond ( 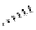 ) are used to represent the absolute configuration of a stereocenter, a straight solid line bond ( 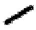 ) and a straight dotted line bond () are used to represent the relative configuration of a stereocenter, a wavy line ( 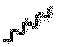 ) may be used to represent a wedge solid line bond ( 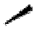 ) or a wedge dotted line bond ( 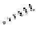 ), or a wavy line ( 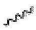 ) may be used to represent a straight solid line bond ( 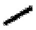 ) or a straight dotted line bond ( 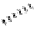 ).

Unless otherwise indicated, the term "enrich in one isomer", "isomerically enriched", "enrich in one enantiomer" or "enantiomerically enriched" means that the content of the one isomer or enantiomer therein is less than 100% but greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise indicated, the term "isomerically excess" or "enantiomerically excess" refers to the difference between the relative percentages of two isomers or two enantiomers. For example, the content of one isomer or enantiomer is 90%, and the content of the other isomer or enantiomer is 10%, then the isomer or enantiomer is 80% excess (ee value).

Optically active (*R*)- and (*S*)-isomers as well as *D* and *L*-isomers may be prepared by chiral synthesis or using chiral reagents or other conventional techniques. To obtain one enantiomer of a compound of the present application, it can be prepared through asymmetric synthesis or derivatization with a chiral auxiliary agent. The resulting diastereomeric mixture is then separated and the auxiliary group is cleaved to provide the pure desired enantiomer. Alternatively, when the molecule contains a basic functional group (such as an amino group) or an acidic functional group (such as a carboxyl group), salts of diastereomers may be formed by reacting with an appropriate optically active acid or base. The diastereomers may then be resolved through conventional methods known in the art, followed by recovery of the pure enantiomers. In addition, the separation of enantiomers and diastereomers is generally achieved by using chromatography in which chiral stationary phases are employed, optionally in combination with chemical derivatization (for example, generation of carbamates from amines).

The compounds of the present application may contain unnatural proportions of atomic isotopes on one or more of the atoms that constitute the compounds. For example, the compounds may be labeled with radioactive isotopes, e.g., tritium (³H), iodine-125 (¹²⁵ I) or C-14 (¹⁴C). Further for example, hydrogen may be replaced by deuterium to form deuterated drugs. The bond between deuterium and carbon is stronger than that between ordinary hydrogen and carbon, and compared with non-deuterated drugs, deuterated drugs have the advantages of reduced toxic and side effects, increased drug stability, enhanced efficacy, and extended biological half-life of drugs. All variations in isotopic composition of the compounds of the present application, whether radioactive or not, are included within the scope of the application.

When the linking group listed does not indicate its linking direction, its linking direction is arbitrary. For example, the linking group L in 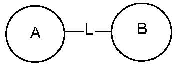 is -M-W-, and -M-W- may either link the ring A and ring B in the same direction as the reading order from left to right to form 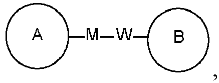 or link the ring A and ring B in the opposite direction to the reading order from left to right to form 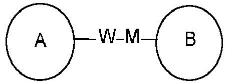 . Combinations of the linking groups, substituents and/or variants thereof are permissible only if such combinations result in stable compounds.

Unless otherwise specified, when a group has one or more linkable sites, any one or more of the sites of the group may be linked to other groups through chemical bonds. If the connection mode of the chemical bond is non-directional and there are hydrogen atoms at the connecting site, when the chemical bond is connected, the number of hydrogen atoms at that site will decrease correspondingly with the number of connected chemical bonds, forming a group of the corresponding valence. The chemical bonds between the site and other groups may be represented by a straight solid line bond ( 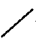 ), a straight dotted line bonds ( 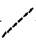 ), or a wavy line ( 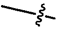 ). For example, the straight solid line bond in -OCH₃ indicates that the group is connected to other groups through the oxygen atom in that group; the straight dotted line bond in 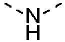 indicates that the group is connected to other groups at the two ends of the nitrogen atom in that group; and the wavy line in 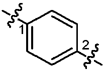 indicates that the phenyl group is connected to other groups through the carbon atoms at positions 1 and 2 of the phenyl group.

Unless otherwise specified, the term "C₁₋₃ alkyl" is used to represent a linear or branched saturated hydrocarbon group compositing of 1 to 3 carbon atoms. The C₁₋₃ alkyl includes C₁₋₂ and C₂₋₃ alkyl, etc., and it may be monovalent (e.g., methyl), divalent (e.g., methylene), or multivalent (e.g., methylidyne). Examples of C₁₋₃ alkyl include, but not limited to, methyl (Me), ethyl (Et), propyl (including n-propyl and isopropyl), etc.

The structure of the compounds of the present application may be determined by conventional methods known to those skilled in the art. If the present application involves the absolute configuration of the compounds, the absolute configuration may be confirmed using conventional techniques in the art. For example, single crystal X-ray diffraction (SXRD) may be used, in which a Bruker D8 venture diffractometer is used to collect diffraction intensity data of the cultured single crystal with CuKα radiation as the light source and in a scanning mode of ϕ/ω scanning, and after collecting relevant data, the crystal structure may be further resolved by a direct method (Shelxs97), thus confirming the absolute configuration.

The compounds of the present application may be prepared by a variety of synthetic methods well known to those skilled in the art, including the specific embodiments listed below, the embodiments resulted from the combination with other chemical synthetic methods, and equivalent alternatives well known to those skilled in the art. The preferred embodiments include, but not limited to, the examples of the present application.

The solvents used herein are commercially available.

The following abbreviations are used herein: aq represents aqueous, eq represents equivalent or equal amount; DCM represents dichloromethane; PE represents petroleum ether; DMSO represents dimethylsulfoxide; MeOH represents methanol; BOC represents tert-butoxycarbonyl, an amine-protecting group; r.t. represents room temperature; O/N represents overnight; THF represents tetrahydrofuran; Boc₂O represents di-tert-butyl dicarbonate; TFA represents trifluoroacetic acid; DIEA represents diisopropylethylamine; DMF represents N,N-dimethylformamide; HBTU represents benzotriazole-N,N,N',N'-tetramethyl-uronium-hexafluorophosphate; HOBT represents 1-hydroxybenzotriazole; HOAT represents 1-hydroxy-7-azabenzotriazole; DIC represents N,N'-diisopropylcarbodiimide; DBU represents 1,8-diazabicyclo[5.4.0]undec-7-ene; PhSiH₃ represents phenylsilane; and Pd(PPh₃)₄ represents tetrakis(triphenylphosphine)palladium.

### DETAILED DESCRIPTION

The present application will be described in detail through examples below, but it does not imply that any unfavorable limitation is imposed on the present application. Although the present application has been described in detail herein where specific embodiments are also disclosed, it is apparent to those skilled in the art that various change and modification may be made to the specific embodiments of the present application without departing from the spirit and scope of the present application.

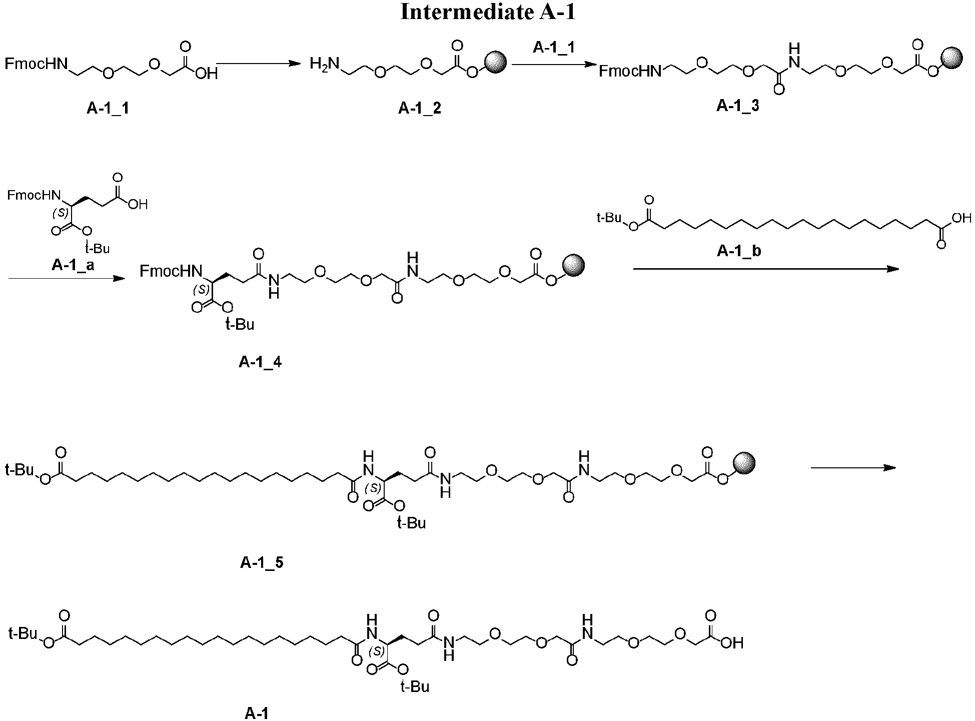

### Step 1: Resin filling

**1.1 4** g of chloro-(o-chlorophenyl)-diphenylmethane resin (degree of substitution S = 1.00 mmol/g) and 1.54 g of **A-1_1** were weighed into a reaction column, into which were further added DCM (25 mL) and then 3 mL of N,N-diisopropylethylamine, and the reaction column was aerated with nitrogen for 2 h. After then, 4 mL of MeOH was added, and the reaction column was continually aerated with nitrogen for 30 minutes. The waste was drained until no liquid flew out. The reaction column was washed with DMF (50 mL) for 5 times, each time for 1 min, and the waste was drained until no liquid flew out.

**1.2** 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 2: Coupling of amino acids

### 2.1 Coupling of A-1_1

1. **A-1_1** (3.0 eq) was weighed and added into the resin, DIEA (6.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
2. The reaction was carried on at 25°C for 20 min. Ninhydrin was used for detection, with the resin appearing colorless and transparent.
3. The reaction solution was drawn off from the reaction column which was then washed with DMF for 5 times, each time for 1 min, and the waste was drained until no liquid flew out.

### 2.2 Coupling of A-1_a

1. 20% of piperidine/DMF (50 mL) was added into the reaction column which was then aerated with nitrogen for 20 min. The waste was drained until no liquid flew out. Ninhydrin was used for detection, with the resin appearing blue.
2. **A-1_a** (3.0 eq) was weighed and added into the resin, DIEA (3.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF (50 mL) for 5 times, each time for 1 min, and the waste was drained until no liquid flew out.

### 2.3 Coupling of A-1_b

1. 20% of piperidine/DMF (50mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. **A-1_b** (3.0 eq) was weighed and added into the resin, DIEA (6.00 eq) plus 20 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times, each time for 1 min, and the waste was drained until no liquid flew out.

### Step 3: Cutting and crude peptide drying

3.1 A cleaving solution was formulated following the volumes below:

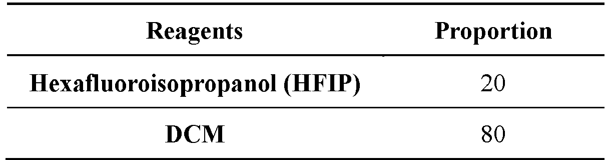

3.2 60 mL of the formulated cleaving solution was poured into a reactor containing the resin with the peptide after being dried. The reactor was aerated for 20 min, the resulted solution was filtered, and the filtrate was added into a flask. The operation was repeated twice, and the collected solution in the two operations was spin-dried to obtain **A-1.**

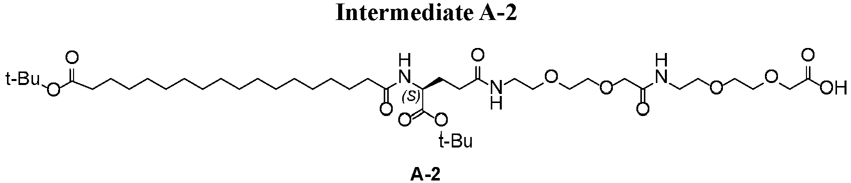

Intermediate A-2 was obtained with reference to the synthesis of Intermediate **A-1.**

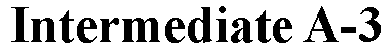

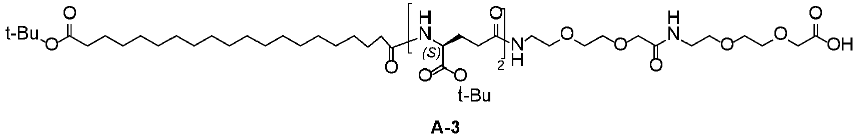

Intermediate A-3 was obtained with reference to the synthesis of Intermediate **A-1.**

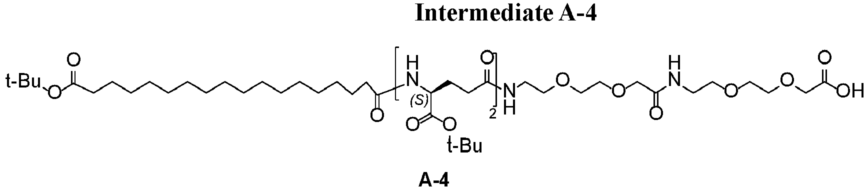

Intermediate A-4 was obtained with reference to the synthesis of Intermediate **A-1.**

### Embodiment 1

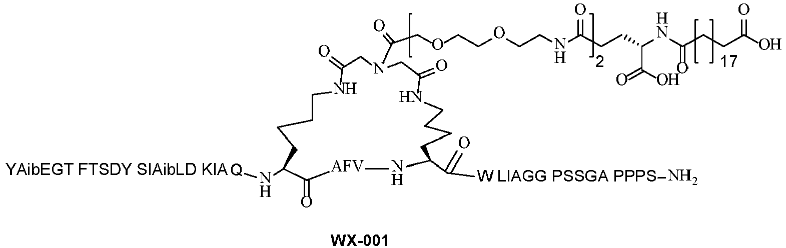

**Step 1:** 1.34 g of 4-(2',4'-dimethoxyphenyl-fluorenemethoxycarbonyl-aminomethyl)-phenoxyacetamido-methylbenzhydrylamine resin (degree of substitution Sub = 0.3 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.

**Step 2:** 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 3: Coupling of amino acids

### 3.1 Coupling of Fmoc-Ser(tBu)-OH

1. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was carried on at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.3 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Chloranil was used for detection, with the resin appearing colorless and transparent.
4. The reaction solution was drawn off from the reaction column which was then washed with DMF for 5 times, each time for 1 min, and the waste was drained until no liquid flew out.

### 3.4 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.5 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.6 Coupling of Fmoc-Gly-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.7 Coupling of Fmoc-Ser(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.8 Coupling of Fmoc-Ser(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.9 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.10 Coupling of Fmoc-Gly-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.11 Coupling of Fmoc-Gly-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.12 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.13 Coupling of Fmoc-Ile-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.14 Coupling of Fmoc-Leu-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.15 Coupling of Fmoc-Trp(Boc)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Trp(Boc)-OH (3.0 eq) was weighed and added into the resin, DIEA(6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.16 Coupling of Fmoc-Lys(Dde)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Dde)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.17 Coupling of Fmoc-Val-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Val-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.18 Coupling of Fmoc-Phe-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.19 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.20 Coupling of Fmoc-Lys(Alloc)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Alloc)-OH (2.0 eq) was weighed and added into the resin, HOBT (2.00 eq) plus 10 mL of DMF were supplemented into the reaction column, which was then aerated with nitrogen and added with DIC (2.00 eq) after the amino acids and HOBT were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.21 Coupling of Fmoc-Gln(Trt)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gln(Trt)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.22 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.23 Coupling of Fmoc-Ile-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.24 Coupling of Fmoc-Lys(Boc)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Boc)-OH (2.0 eq) was weighed and added into the resin, DIEA(6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.25 Coupling of Fmoc-Asp(OtBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.26 Coupling of Fmoc-Leu-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.27 Coupling of Fmoc-Aib-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min and the waste was drained until no liquid flew out. Ninhydrin was used for detection, with the resin appearing blue.
2. Fmoc-Aib-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 2 h. Ninhydrin was used for detection, with the resin appearing blue.
4. The reaction solution was drawn off from the reaction column which was then washed with DMF for 5 times (50 mL each time), each time for 1 min, and the waste was drained until no liquid flew out.

### 3.28 Coupling of Fmoc-Ile-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (6.0 eq) was weighed and added into the resin, HOAT (6.00 eq) plus 10 mL of DMF were supplemented into the reaction column, which was then aerated with nitrogen and added with DIC (6.00 eq) after the amino acids and HOAT were dissolved. Nitrogen was adjusted to make the resin bulge evenly.
3. The reaction was carried on at 25°C for 1 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.29 Coupling of Fmoc-Ser(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.30 Coupling of Fmoc-Tyr(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Tyr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.31 Coupling of Fmoc-Asp(OtBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.32 Coupling of Fmoc-Ser(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.33 Coupling of Fmoc-Thr(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.34 Coupling of Fmoc-Phe-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.35 Coupling of Fmoc-Thr(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.36 Coupling of Fmoc-Gly-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.0 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.37 Coupling of Fmoc-Glu(OtBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Glu(OtBu)-OH (6.0 eq) was weighed and added into the resin, HOBT (6.00 eq) plus 10 mL of DMF were supplemented into the reaction column, which was then aerated with nitrogen and added with DIC (6.00 eq) after the amino acids and HOBT were dissolved. Nitrogen was adjusted to make the resin bulge evenly.
3. The reaction was carried on at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.38 Coupling of Fmoc-Aib-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Aib-OH (3.0 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried on at 25°C overnight. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.39 Coupling of Boc-Tyr(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Boc-Tyr(tBu)-OH (6.0 eq) was weighed and added into the resin, DIEA (12.0 eq) plus 10 mL of DMF were supplemented into the reaction column, which was then aerated with nitrogen and added with HATU (5.70 eq) after the amino acids were dissolved. Nitrogen was adjusted to make the resin bulge evenly.
3. The reaction was carried on at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.40 Removal of Alloc

1. PhSiH₃ (10.0 eq) and DCM (10 mL) were added into the reaction column, which was then aerated with nitrogen and added with Pd(PPh₃)₄ (0.1 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.41 Coupling of Fmoc-Ida-OH

1. Fmoc-Ida-OH (4. 0 eq) was weighed and added into the resin, DIEA (8.00 eq) plus 10 mL of DMF were supplemented into the reaction column, which was then aerated with nitrogen and added with HBTU (3.80 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.42 Coupling of Intermediate A-1

1. 10% of DBU in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HBTU (1.45 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.43 Removal of Dde

1. 3% of hydrazine/DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.44 Amide ring closing

1. DIEA (3.0 eq) in DMF was added into the resin, then a solution of HATU (1.5 eq) in DMF was dropwise added into the reaction column slowly, which was then aerated with nitrogen. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below: 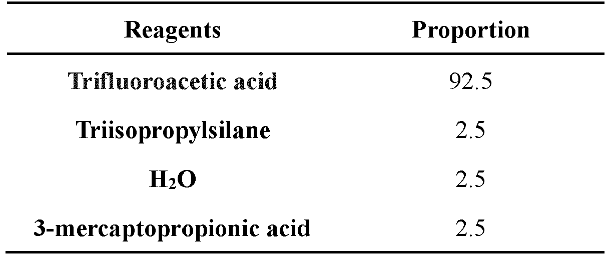
4.2 The dried resin with the peptide was added into the formulated cleaving solution, shaken on a shaker for 2.5 h, and filtered. The filtrate was added into 10 times the volume of ice-cold isopropyl ether, centrifuged, and washed for 5 times with isopropyl ether. It was then dried in vacuum for 2 h to get a crude peptide, which was purified to get a polypeptide compound **WX-001.** The molecular weight of the polypeptide was confirmed by ESI-MS, with a calculated value of [M+4H]/4 as 1228.6 and a detected value of 1228.7.

### Embodiment 2

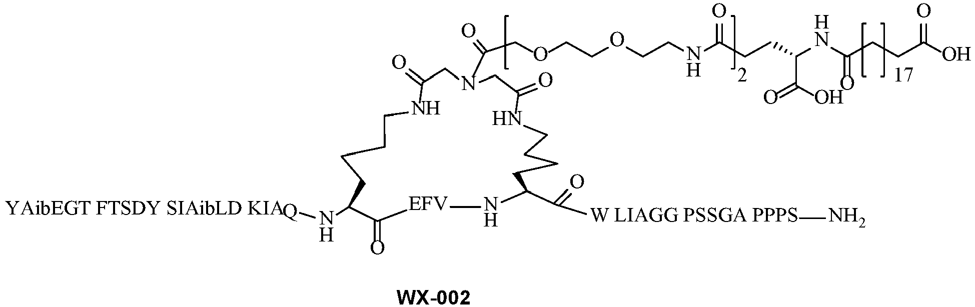

With reference to the synthesis of **WX-001,** a polypeptide **WX-002** was obtained. The molecular weight of the polypeptide was confirmed by ESI-MS, with a calculated value of [M+4H]/4 as 1243.1 and a detected value of 1242.8.

### Embodiment 3

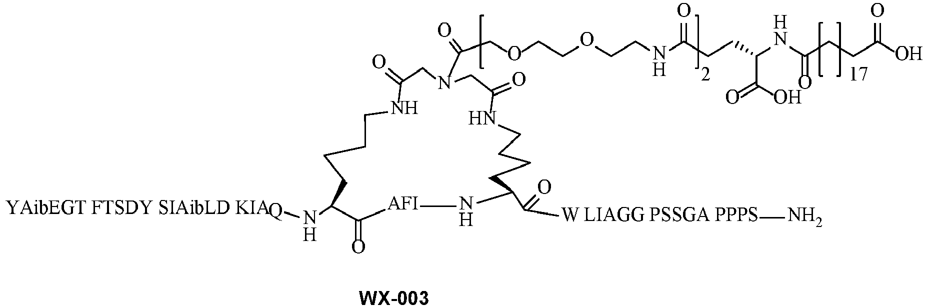

With reference to the synthesis of **WX-001,** a polypeptide **WX-003** was obtained. The molecular weight of the polypeptide was confirmed by ESI-MS, with a calculated value of [M+4H]/4 as 1232.1 and a detected value of 1232.2.

### Embodiment 4

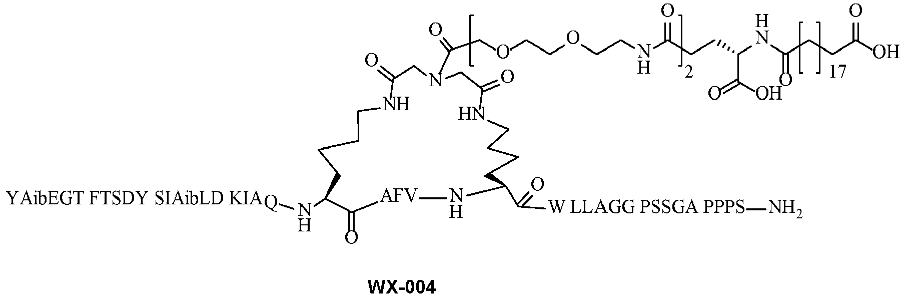

With reference to the synthesis of **WX-001,** a polypeptide **WX-004** was obtained. The molecular weight of the polypeptide was confirmed by ESI-MS, with a calculated value of [M+4H]/4 as 1228.6 and a detected value of 1228.2.

### Embodiment 5

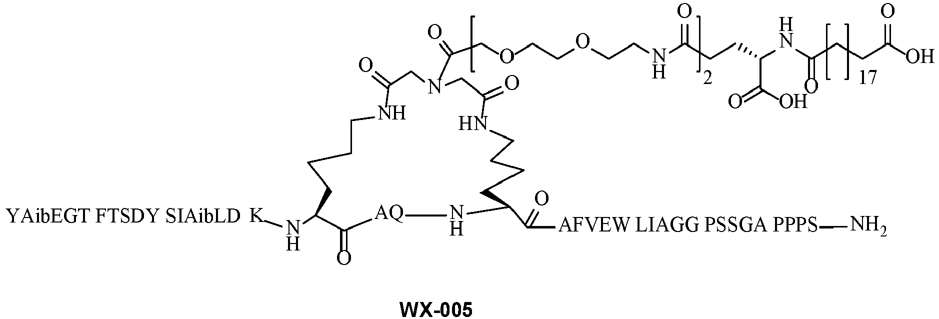

**Step 1:** 1.34 g of 4-(2',4'-dimethoxyphenyl-fluorenemethoxycarbonyl-aminomethyl)-phenoxyacetamido-methylbenzhydrylamine resin (degree of substitution Sub = 0.3 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.

**Step 2:** 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.

### Step 3: Coupling of amino acids

### 3.1 Coupling of Fmoc-Ser(tBu)-OH

1. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

**5. Step 3.2 was similarly repeated to perform the coupling of the following amino acids.**

| **No.** | **Raw materials** | **Coupling reagents** |
|---|---|---|
| 3.3 | Fmoc-Pro-OH (3.00 eq) | HBTU (2.85 eq) and DIEA (6.00 eq) |
| 3.4 | Fmoc-Pro-OH (3.00 eq) | HBTU (2.85 eq) and DIEA (6.00 eq) |
| 3.5 | Fmoc-Ala-OH (3.00 eq) | HBTU (2.85 eq) and DIEA (6.00 eq) |
| 3.6 | Fmoc-Gly-OH (3.00 eq) | HBTU (2.85 eq) and DIEA (6.00 eq) |
| 3.7 | Fmoc-Ser(tBu)-OH (3.00 eq) | HBTU (2.85 eq) and DIEA (6.00 eq) |
| 3.8 | Fmoc-Ser(tBu)-OH (3.00 eq) | HBTU (2.85 eq) and DIEA (6.00 eq) |
| 3.9 | Fmoc-Pro-OH (3.00 eq) | HBTU (2.85 eq) and DIEA (6.00 eq) |
| 3.10 | Fmoc-Gly-OH (3.00 eq) | HBTU (2.85 eq) and DIEA (6.00 eq) |
| 3.11 | Fmoc-Gly-OH (3.00 eq) | HBTU (2.85 eq) and DIEA (6.00 eq) |
| 3.12 | Fmoc-Ala-OH (3.00 eq) | HBTU (2.85 eq) and DIEA (6.00 eq) |
| 3.13 | Fmoc-Ile-OH (3.00 eq) | HBTU (2.85 eq) and DIEA (6.00 eq) |
| 3.14 | Fmoc-Leu-OH (3.00 eq) | HBTU (2.85 eq) and DIEA (6.00 eq) |
| 3.15 | Fmoc-Trp(Boc)-OH (3.00 eq) | HBTU (2.85 eq) and DIEA (6.00 eq) |
| 3.16 | Fmoc-Glu(tBu)-OH (2.00 eq) | HBTU (1.90 eq) and DIEA (4.00 eq) |
| 3.17 | Fmoc-Val-OH (3.00 eq) | HBTU (2.85 eq) and DIEA (6.00 eq) |
| 3.18 | Fmoc-Phe-OH (3.00 eq) | HBTU (2.85 eq) and DIEA (6.00 eq) |
| 3.19 | Fmoc-Ala-OH (3.00 eq) | HBTU (2.85 eq) and DIEA (6.00 eq) |
| 3.20 | Fmoc-Lys(Dde)-OH(3.00 eq) | HOBT (2.00 eq) and DIC (2.00 eq) |
| 3.21 | Fmoc-Gln(Trt)-OH (3.00 eq) | HBTU (2.85 eq) and DIEA (6.00 eq) |
| 3.22 | Fmoc-Ala-OH (3.00 eq) | HBTU (2.85 eq) and DIEA (6.00 eq) |
| 3.23 | Fmoc-Lys(Alloc)-OH (2.00 eq) | HBTU (2.85 eq) and DIEA (6.00 eq) |
| 3.24 | Fmoc-Lys(Boc)-OH (3.00 eq) | HBTU (2.85 eq) and DIEA (6.00 eq) |
| 3.25 | Fmoc-Asp(tBu)-OH (6.00 eq) | HATU (5.70 eq) and DIEA (12.0 eq) |
| 3.26 | Fmoc-Leu-OH (6.00 eq) | HATU (5.70 eq) and DIEA (12.0 eq) |
| 3.27 | Fmoc-Aib-OH (6.00 eq) | HATU (5.70 eq) and DIEA (12.0 eq) |
| 3.28 | Fmoc-Ile-OH (6.00 eq) | HOAT (6.00 eq) and DIC (6.00 eq) |
| 3.29 | Fmoc-Ser(tBu)-OH (6.00 eq) | HATU (5.70 eq) and DIEA (12.0 eq) |
| 3.30 | Fmoc-Tyr(tBu)-OH (6.00 eq) | HATU (5.70 eq) and DIEA (12.0 eq) |
| 3.31 | Fmoc-Asp(tBu)-OH (6.00 eq) | HATU (5.70 eq) and DIEA (12.0 eq) |
| 3.32 | Fmoc-Ser(tBu)-OH (6.00 eq) | HATU (5.70 eq) and DIEA (12.0 eq) |
| 3.33 | Fmoc-Thr(tBu)-OH (6.00 eq) | HATU (5.70 eq) and DIEA (12.0 eq) |
| 3.34 | Fmoc-Phe-OH (6.00 eq) | HATU (5.70 eq) and DIEA (12.0 eq) |
| 3.35 | Fmoc-Thr(tBu)-OH (6.00 eq) | HATU (5.70 eq) and DIEA (12.0 eq) |
| 3.36 | Fmoc-Gly-OH (6.00 eq) | HATU (5.70 eq) and DIEA (12.0 eq) |
| 3.37 | Fmoc-Glu(tBu)-OH (6.00 eq) | HOBT (6.00 eq) and DIC (6.00 eq) |
| 3.38 | Fmoc-Aib-OH (3.00 eq) | HATU (2.85 eq) and DIEA (6.00 eq) |
| 3.39 | Boc-Tyr(tBu)-OH (3.00 eq) | HATU (2.85 eq) and DIEA (6.00 eq) |

### 3.40 Removal of Alloc

1. PhSiH₃ (10.0 eq) and DCM (10 mL) were added into the reaction column, which was then aerated with nitrogen and added with Pd(PPh₃)₄ (0.1 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.41 Coupling of Fmoc-Ida-OH

1. Fmoc-Ida-OH (4. 0 eq) was weighed and added into the resin, DIEA (8.00 eq) plus 10 mL of DMF were supplemented into the reaction column, which was then aerated with nitrogen and added with HBTU (3.80 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.42 Coupling of Intermediate A-1

1. 10% of DBU in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HBTU (1.45 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.43 Removal of Dde

1. 3% of hydrazine/DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.44 Amide ring closing

1. DIEA (3.0 eq) in DMF was added into the resin, then a solution of HATU (1.5 eq) in DMF was dropwise added into the reaction column slowly, which was then aerated with nitrogen. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below:

| **Reagents** | **Proportion** |
|---|---|
| **Trifluoroacetic acid** | 92.5 |
| **Triisopropylsilane** | 2.5 |
| **H₂O** | 2.5 |
| **3-mercaptopropionic acid** | 2.5 |

4.2 The dried resin with the peptide was added into the formulated cleaving solution, shaken on a shaker for 2.5 h, and filtered. The filtrate was added into 10 times the volume of ice-cold isopropyl ether, centrifuged, and washed for 5 times with isopropyl ether. It was then dried in vacuum for 2 h to get a crude peptide, which was purified to get a polypeptide **WX-005.** The molecular weight of the polypeptide was confirmed by ESI-MS, with a calculated value of [M+4H]/4 as 1232.6 and a detected value of 1232.5.

### Embodiment 6

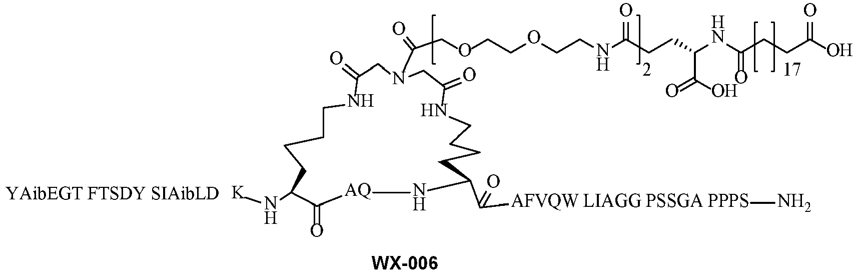

With reference to the synthesis of **WX-001,** a polypeptide **WX-006** was obtained. The molecular weight of the polypeptide was confirmed by ESI-MS, with a calculated value of [M+4H]/4 as 1232.4 and a detected value of 1232.4.

### Embodiment 7

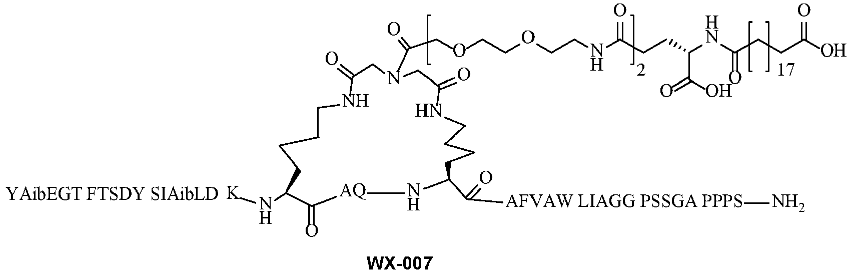

With reference to the synthesis of **WX-001,** a polypeptide **WX-007** was obtained. The molecular weight of the polypeptide was confirmed by ESI-MS, with a calculated value of [M+4H]/4 as 1218.1 and a detected value of 1218.3.

### Embodiment 8

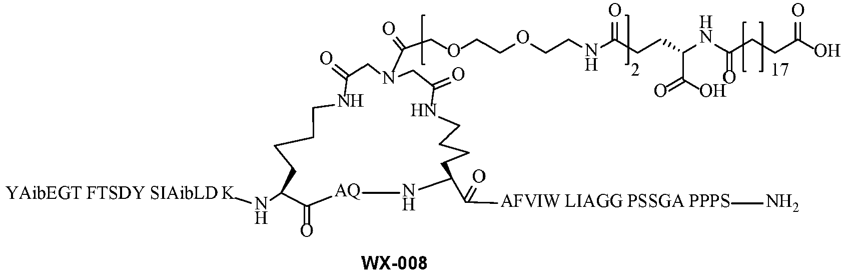

With reference to the synthesis of **WX-001,** a polypeptide **WX-008** was obtained. The molecular weight of the polypeptide was confirmed by ESI-MS, with a calculated value of [M+4H]/4 as 1228.6 and a detected value of 1228.6.

### Embodiment 9

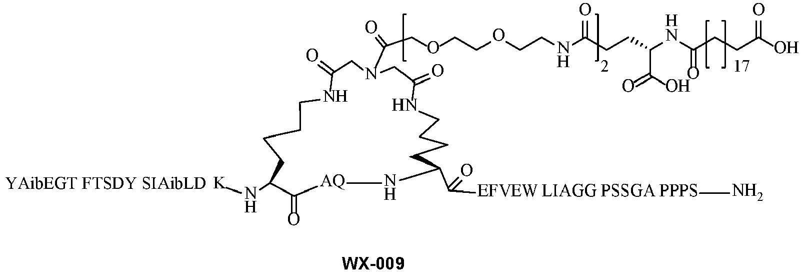

**Step 1:** 1.34 g of 4-(2',4'-dimethoxyphenyl-fluorenemethoxycarbonyl-aminomethyl)-phenoxyacetamido-methylbenzhydrylamine resin (degree of substitution Sub = 0.3 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.
**Step 2:** 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
**Step 3: Coupling of amino acids**

### 3.1 Coupling of Fmoc-Ser(tBu)-OH

1. Fmoc-Ser(tBu)-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.0 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

5. **Step 3.2 was similarly repeated to perform the coupling of the following amino acids.**

| **No.** | **Raw materials** | **Coupling reagents** |
|---|---|---|
| 3.3 | Fmoc-Pro-OH (3.00 eq) | HBTU (2.85 eq) and DIEA (6.00 eq) |
| 3.4 | Fmoc-Pro-OH (3.00 eq) | HBTU (2.85 eq) and DIEA (6.00 eq) |
| 3.5 | Fmoc-Ala-OH (3.00 eq) | HBTU (2.85 eq) and DIEA (6.00 eq) |
| 3.6 | Fmoc-Gly-OH (3.00 eq) | HBTU (2.85 eq) and DIEA (6.00 eq) |
| 3.7 | Fmoc-Ser(tBu)-OH (3.00 eq) | HBTU (2.85 eq) and DIEA (6.00 eq) |
| 3.8 | Fmoc-Ser(tBu)-OH (3.00 eq) | HBTU (2.85 eq) and DIEA (6.00 eq) |
| 3.9 | Fmoc-Pro-OH (3.00 eq) | HBTU (2.85 eq) and DIEA (6.00 eq) |
| 3.10 | Fmoc-Gly-OH (3.00 eq) | HBTU (2.85 eq) and DIEA (6.00 eq) |
| 3.11 | Fmoc-Gly-OH (3.00 eq) | HBTU (2.85 eq) and DIEA (6.00 eq) |
| 3.12 | Fmoc-Ala-OH (3.00 eq) | HBTU (2.85 eq) and DIEA (6.00 eq) |
| 3.13 | Fmoc-Ile-OH (3.00 eq) | HBTU (2.85 eq) and DIEA (6.00 eq) |
| 3.14 | Fmoc-Leu-OH (3.00 eq) | HBTU (2.85 eq) and DIEA (6.00 eq) |
| 3.15 | Fmoc-Trp(Boc)-OH (3.00 eq) | HBTU (2.85 eq) and DIEA (6.00 eq) |
| 3.16 | Fmoc-Glu(tBu)-OH (2.00 eq) | HBTU (1.90 eq) and DIEA (4.00 eq) |
| 3.17 | Fmoc-Val-OH (3.00 eq) | HBTU (2.85 eq) and DIEA (6.00 eq) |
| 3.18 | Fmoc-Phe-OH (3.00 eq) | HBTU (2.85 eq) and DIEA (6.00 eq) |
| 3.19 | Fmoc-Glu(tBu)-OH (6.00 eq) | HBTU (2.85 eq) and DIEA (6.00 eq) |
| 3.20 | Fmoc-Lys(Dde)-OH(3.00 eq) | HOBT (2.00 eq) and DIC (2.00 eq) |
| 3.21 | Fmoc-Gln(Trt)-OH (3.00 eq) | HBTU (2.85 eq) and DIEA (6.00 eq) |
| 3.22 | Fmoc-Ala-OH (3.00 eq) | HBTU (2.85 eq) and DIEA (6.00 eq) |
| 3.23 | Fmoc-Lys(Alloc)-OH (2.00 eq) | HBTU (2.85 eq) and DIEA (6.00 eq) |
| 3.24 | Fmoc-Lys(Boc)-OH (3.00 eq) | HBTU (2.85 eq) and DIEA (6.00 eq) |
| 3.25 | Fmoc-Asp(tBu)-OH (6.00 eq) | HATU (5.70 eq) and DIEA (12.0 eq) |
| 3.26 | Fmoc-Leu-OH (6.00 eq) | HATU (5.70 eq) and DIEA (12.0 eq) |
| 3.27 | Fmoc-Aib-OH (6.00 eq) | HATU (5.70 eq) and DIEA (12.0 eq) |
| 3.28 | Fmoc-Ile-OH (6.00 eq) | HOAT (6.00 eq) and DIC (6.00 eq) |
| 3.29 | Fmoc-Ser(tBu)-OH (6.00 eq) | HATU (5.70 eq) and DIEA (12.0 eq) |
| 3.30 | Fmoc-Tyr(tBu)-OH (6.00 eq) | HATU (5.70 eq) and DIEA (12.0 eq) |
| 3.31 | Fmoc-Asp(tBu)-OH (6.00 eq) | HATU (5.70 eq) and DIEA (12.0 eq) |
| 3.32 | Fmoc-Ser(tBu)-OH (6.00 eq) | HATU (5.70 eq) and DIEA (12.0 eq) |
| 3.33 | Fmoc-Thr(tBu)-OH (6.00 eq) | HATU (5.70 eq) and DIEA (12.0 eq) |
| 3.34 | Fmoc-Phe-OH (6.00 eq) | HATU (5.70 eq) and DIEA (12.0 eq) |
| 3.35 | Fmoc-Thr(tBu)-OH (6.00 eq) | HATU (5.70 eq) and DIEA (12.0 eq) |
| 3.36 | Fmoc-Gly-OH (6.00 eq) | HATU (5.70 eq) and DIEA (12.0 eq) |
| 3.37 | Fmoc-Glu(tBu)-OH (6.00 eq) | HOBT (6.00 eq) and DIC (6.00 eq) |
| 3.38 | Fmoc-Aib-OH (3.00 eq) | HATU (2.85 eq) and DIEA (6.00 eq) |
| 3.39 | Boc-Tyr(tBu)-OH (3.00 eq) | HATU (2.85 eq) and DIEA (6.00 eq) |

### 3.40 Removal of Alloc

1. PhSiH₃ (10.0 eq) and DCM (10 mL) were added into the reaction column, which was then aerated with nitrogen and added with Pd(PPh₃)₄ (0.1 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.41 Coupling of Fmoc-Ida-OH

1. Fmoc-Ida-OH (4. 0 eq) was weighed and added into the resin, DIEA (8.00 eq) plus 10 mL of DMF were supplemented into the reaction column, which was then aerated with nitrogen and added with HBTU (3.80 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.42 Coupling of Intermediate A-1

1. 10% of DBU in DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HBTU (1.45 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.43 Removal of Dde

1. 3% of hydrazine/DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.44 Amide ring closing

1. DIEA (3.0 eq) in DMF was added into the resin, then a solution of HATU (1.5 eq) in DMF was dropwise added into the reaction column slowly, which was then aerated with nitrogen. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below:

| **Reagents** | **Proportion** |
|---|---|
| **Trifluoroacetic acid** | 92.5 |
| **Triisopropylsilane** | 2.5 |
| **H₂O** | 2.5 |
| **3-mercaptopropionic acid** | 2.5 |

4.2 The peptide resin with the peptide was added into the formulated cleaving solution, shaken on a shaker for 2.5 h, and filtered. The filtrate was added into 10 times the volume of ice-cold isopropyl ether, centrifuged, and washed for 5 times with isopropyl ether. It was then dried in vacuum for 2 h to get a crude peptide, which was purified to get a polypeptide **WX-009.** The molecular weight of the polypeptide was confirmed by ESI-MS, with a calculated value of [M+4H]/4 as 1247.1 and a detected value of 1247.2.

### Embodiment 10

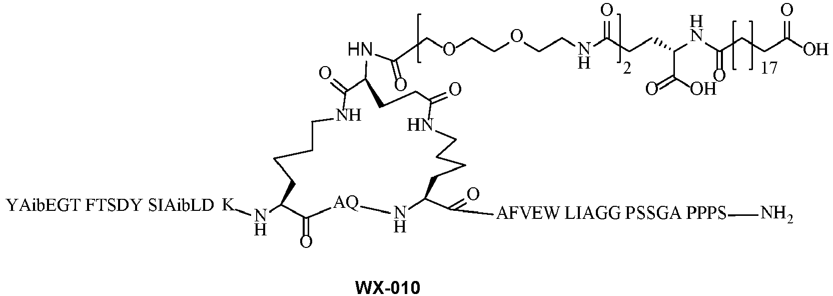

**Step 1:** 1.08 g of 4-(2',4'-dimethoxyphenyl-fluorenemethoxycarbonyl-aminomethyl)-phenoxyacetamido-methylbenzhydrylamine resin (degree of substitution Sub = 0.37 mmol/g) was weighed and added into a reaction column, into which was further added DMF (50 mL), and the reaction column was aerated with nitrogen for 2 h. After then, the waste was drained until no liquid flew out. The reaction column was washed with DMF (50 mL) for 5 times, each time for 1 min, and the waste was drained until no liquid flew out.
**Step 2:** 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min and the waste was drained until no liquid flew out. Ninhydrin was used for detection, with the resin appearing blue.
**Step 3: Coupling of amino acids**

### 3.1 Coupling of Fmoc-Ser(tBu)-OH

1. Fmoc-Ser(tBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.2 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.3 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.4 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.5 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.6 Coupling of Fmoc-Gly-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.7 Coupling of Fmoc-Ser(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.8 Coupling of Fmoc-Ser(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.9 Coupling of Fmoc-Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Pro-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.10 Coupling of Fmoc-Gly-Gly-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Fmoc-Gly-Gly-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.11 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.12 Coupling of Fmoc-Ile-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.13 Coupling of Fmoc-Leu-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.14 Coupling of Fmoc-Trp(Boc)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Trp(Boc)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.15 Coupling of Fmoc-Glu(OtBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Glu(OtBu)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.16 Coupling of Fmoc-Val-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Val-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.17 Coupling of Fmoc-Phe-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.18 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.19 Coupling of Fmoc-Lys(Alloc)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Alloc)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.20 Coupling of Fmoc-Gln(Trt)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gln(Trt)-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.21 Coupling of Fmoc-Ala-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ala-OH (3.00 eq) was weighed and added into the resin, DIEA (6.00 eq) plus 10 mL of DMF were supplemented into the reaction column, which was then aerated with nitrogen and added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.22 Coupling of Fmoc-Lys(Dde)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Dde)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.23 Coupling of Fmoc-Lys(Boc)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Lys(Boc)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.24 Coupling of Fmoc-Asp(OtBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.25 Coupling of Fmoc-Leu-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Leu-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.26 Coupling of Fmoc-Aib-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min and the waste was drained until no liquid flew out. Ninhydrin was used for detection, with the resin appearing blue.
2. Fmoc-Aib-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 2 h. Ninhydrin was used for detection, with the resin appearing blue.
4. The reaction solution was drawn off from the reaction column which was then washed with DMF for 5 times (50 mL each time), each time for 1 min, and the waste was drained until no liquid flew out.

### 3.27 Coupling of Fmoc-Ile-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ile-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 1 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.28 Coupling of Fmoc-Ser(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Ser(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.29 Coupling of Fmoc-Tyr(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Tyr(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.30 Coupling of Fmoc-Asp(OtBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Asp(OtBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.31 Coupling of Fmoc-Thr(tBu)-SerPsi(Me,Me)Pro-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-SerPsi(Me,Me)Pro-OH (2.00 eq) was weighed and added into the resin, DIEA (4.00 eq) plus 10 mL of DMF were supplemented into the reaction column, which was then aerated with nitrogen and added with HATU (1.90 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.32 Coupling of Fmoc-Phe-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Phe-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.33 Coupling of Fmoc-Thr(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Thr(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.34 Coupling of Fmoc-Gly-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Gly-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.35 Coupling of Fmoc-Glu(OtBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Glu(OtBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.36 Coupling of Fmoc-Aib-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. Fmoc-Aib-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C overnight. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.37 Coupling of Boc-Tyr(tBu)-OH

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Chloranil was used for detection, with the resin appearing blue.
2. Boc-Tyr(tBu)-OH (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
3. The reaction was carried out at 25°C for 0.5 h. Chloranil was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.38 Removal of Alloc

1. PhSiH₃ (10.0 eq) and DCM (20 mL) were added into the reaction column, which was then aerated with nitrogen and added with Pd(PPh₃)₄ (0.10 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.39 Coupling of Fmoc-Glu-OAll

1. Fmoc-Glu-OAll (3.00 eq) was weighed and added into the resin, DIEA (6.00 eq) plus 10 mL of DMF were supplemented into the reaction column, which was then aerated with nitrogen and added with HATU (2.85 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.

### 3.40 Coupling of Intermediate A-1

1. 20% of piperidine/DMF (50 mL) was added into the reaction column which was then aerated with nitrogen for 20 min. The waste was drained until no liquid flew out. The reaction column was washed with DMF (50 mL) for 5 times, each time for 1 min, and the waste was drained until no liquid flew out. Ninhydrin was used for detection, with the resin appearing blue.
2. **Intermediate A-1** (1.50 eq) was weighed and added into the resin, DIEA (3.00 eq) plus 10 mL of DMF were supplemented into the reaction column, which was then aerated with nitrogen and added with HATU (1.45 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off from the reaction column which was then washed with DMF for 5 times (50 mL each time), each time for 1 min, and the waste was drained until no liquid flew out.

### 3.41 Removal of Alloc

1. PhSiH₃ (10.0 eq) and DCM (20 mL) were added into the reaction column, which was then aerated with nitrogen and added with Pd(PPh₃)₄ (0.10 eq), then aerated with nitrogen for 20 min. The reaction was carried out twice, and the waste was drained until no liquid flew out.
2. The reaction column was washed with DMF for 5 times (50 mL each time), each time for 1 min, and the waste was drained until no liquid flew out.

### 3.42 Removal of Dde

1. 3% of hydrazine hydrate/DMF (50 mL) was added into the reaction column which was then aerated with nitrogen for 15 min, and the waste was drained. The reaction column was washed with DMF (50 mL) for 5 times, each time for 1 min, and the waste was drained until no liquid flew out. Ninhydrin was used for detection, with the resin appearing blue.

### 3.42 Amide ring closing

1. DIEA (3.00 eq) in DMF was added into the resin, then a solution of HATU (1.50 eq) in DMF was dropwise added into the reaction column slowly, which was then aerated with nitrogen. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off from the reaction column which was then washed with DMF for 5 times (50 mL each time), each time for 1 min, and the waste was drained until no liquid flew out.
4. The resin was shrunk with MeOH (50 mL) for 3 min each time. The waste was drained until no liquid flew out. The resin was poured out and dried for further use.

### Step 4: Cutting and crude peptide drying

4.1 A cleaving solution was formulated following the volumes below:

| Reagents | Proportion |
|---|---|
| **Trifluoroacetic acid** | 92.5 |
| **Triisopropylsilane** | 2.5 |
| **H₂O** | 2.5 |
| **3-mercaptopropionic acid** | 2.5 |

4.2 The dried resin with the peptide was added into the formulated cleaving solution, shaken on a shaker for 2.5 h, and filtered. The filtrate was added into 10 times the volume of ice-cold isopropyl ether, centrifuged, and washed for 5 times with isopropyl ether. It was then dried in vacuum for 2 h to get a crude peptide, which was purified. The molecular weight of the polypeptide was confirmed by ESI-MS, with a calculated value of as 1236.1 and a detected value of 1236.0.

### Embodiment 11

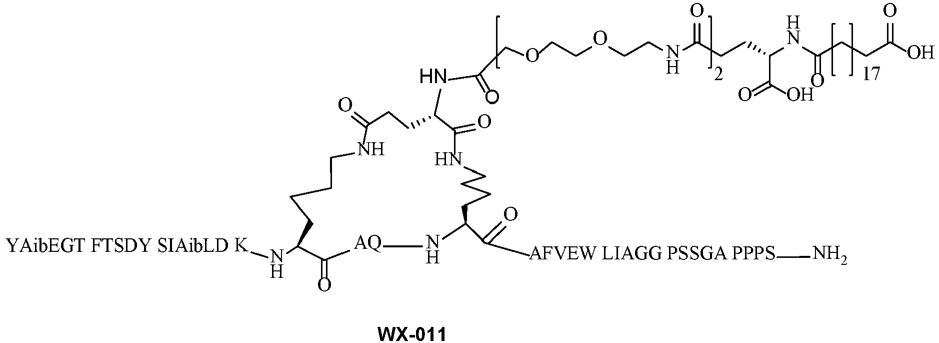

With reference to the synthesis of **WX-010,** a polypeptide **WX-011** was obtained. The molecular weight of the polypeptide was confirmed by ESI-MS, with a calculated value of [M+4H]/4 as 1236.1 and a detected value of 1236.0.

### Embodiment 12

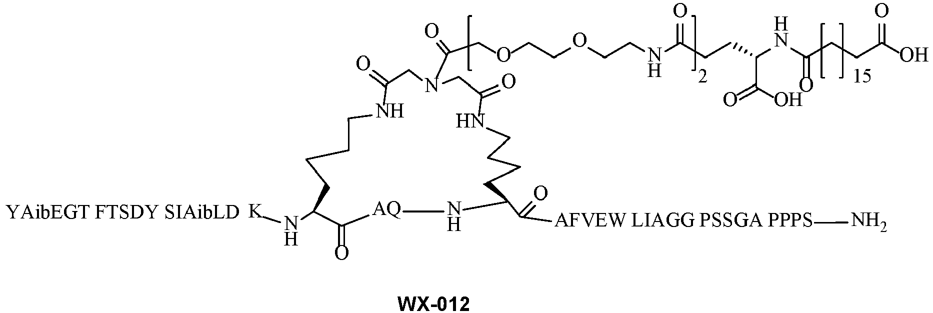

With reference to the synthesis of **WX-001,** a polypeptide **WX-012** was obtained using the intermediate A-2. The molecular weight of the polypeptide was confirmed by ESI-MS, with a calculated value of [M+4H]/4 as 1225.6 and a detected value of 1225.7.

### Embodiment 13

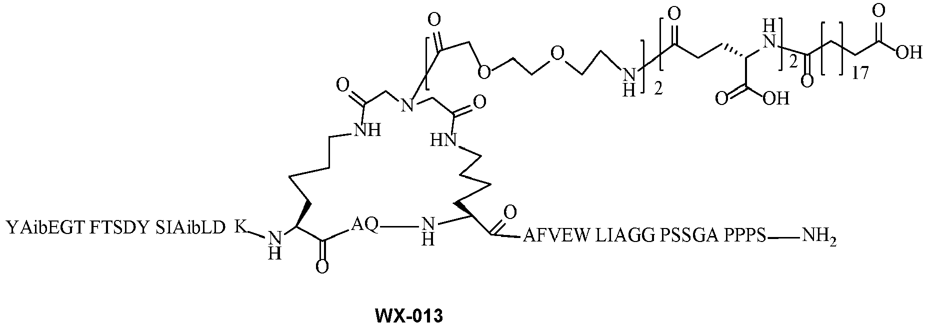

With reference to the synthesis of **WX-001** and using the intermediate **A-3,** a polypeptide **WX-013** was obtained. The molecular weight of the polypeptide was confirmed by ESI-MS, with a calculated value of [M+4H]/4 as 1264.9 and a detected value of 1264.6.

### Embodiment 14

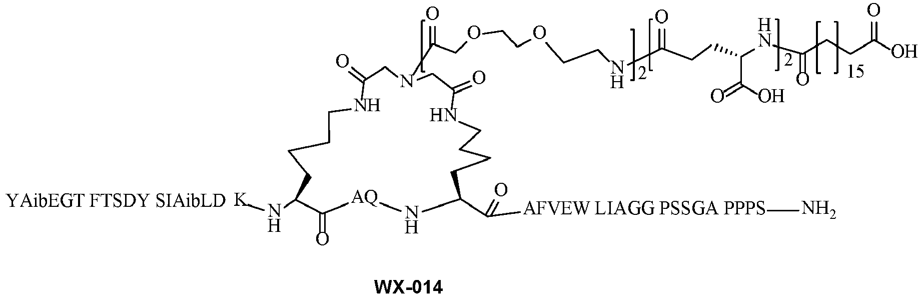

With reference to the synthesis of **WX-001** and using the intermediate **A-4,** a polypeptide **WX-014** was obtained. The molecular weight of the polypeptide was confirmed by ESI-MS, with a calculated value of [M+4H]/4 as 1257.9 and a detected value of 1257.8.

### Biological test data

### Test example 1: In vitro GLP-1R/GIPR agonistic activity test

### A. Main Materials:

### 1) Cell lines

The cell lines were constructed by Wuxi APPTEC (Shanghai) Co., Ltd. See details in Table 1 below.

**Table 1 Cell Lines Information**

| Target | Host cell | Clone |
|---|---|---|
| GLP-1R | HEK293 | N/A |
| GIPR | CHO | N/A |

### 2) Reagents and Consumables

**Table 2 Reagents and Consumables Information**

| Name | Lot No. | Item No. | Manufacturer |
|---|---|---|---|
| cAMP kit | 29F | 62AM4PEJ | Cisbio |
| 1M HEPES | 2120919 | 15630-106 | Invitrogen |
| Hanks' Balanced Salt Solution (HBSS) | 2185775 | 14025 | Invitrogen |
| Human serum albumin (HSA) | SLCF7301 | A1653-10G | Sigma |

| | | | |
|---|---|---|---|
| Casein | SLCC9458 | C4765-10 mL | Sigma |
| 3-isobutyl-1-methylxanthine (IBMX) | STBF6061V | I5879-5G | Sigma |
| ECHO qualified 384-well plate | 0006433672 | PP-0200 | Labcyte |
| OptiPlate-384 | 8210-19481 | 6007299 | PerkinElmer |

### 3) Instruments

**Table 3 Instruments Information**

| Name | Model | Manufacturer |
|---|---|---|
| EnVision | envision2014 | PerkinElmer |
| Vi-cell counter | Vi-CELL^{™} XR Cell Viability Analyzer | Beckman |
| Bravo | Bravo V11 | Agilent |
| ECHO | ECHO 555 | Labcyte |
| Centrifuge | Allegra^{™} 25R Centrifuge | Beckman |

### B. Methods

### 1) Experimental Materials

### Experimental buffers

**Table 4 Buffers Information**

| **Reagents** | **Storage concentration** | **Volume** | **Final concentration** |
|---|---|---|---|
| Hanks' Balanced Salt Solution | 1× | 48.7 mL/44.7 mL | ≈1 × |
| HEPES buffer | 1 mol/L | 250 µL | 5 mmol/L |
| 5% of casein solution (HEPES)/ 10% of human serum albumin solution (HSA) | 5%/ 10% | 1000 µL/ 5000 µL | 0.10%/ 1% |
| 3 -isobutyl-1-methylxanthine (IBMX) | 500 mmol/L | 50 µL | 0.5 mmol/L |

### Preparation of assay reagents

**Table 5 Assay Reagents Information**

| **Reagents** | **Storage concentration** | **Volume** | **Final concentration** |
|---|---|---|---|
| Cell lysis buffer | 1× | 9.5 mL | ≈1× |
| D2-cAMP solution | 40× | 250 µL | 1× |
| cAMP-antibody solution | 40× | 250 µL | 1× |

### 2) Experimental Methods

a) Preparation of compound plates:
   The test compounds were diluted at a 4-fold dilution factor in 10 points, starting from an initial concentration of 30 µM. The dilution was carried out by Bravo.
b) Transfer of compounds:
   1) 100 nL of compounds were transferred to an OptiPlate-384 plate by Echo.
   2) The OptiPlate-384 plate was centrifuged at 1000 rpm for 5 s.
c) Preparation of cell suspension:
   1) A GLP-1R/GIPR cell cryovial was placed in warm water at 37°C for rapid thawing.
   2) The cell suspension was transferred into a 15 mL Transfer centrifugal tube and rinsed with 10 mL of HBSS gently.
   3) The centrifugal tube was centrifuged at 1000 rpm at room temperature for 1 min.
   4) The supernatant was discarded.
   5) The bottom cells were gently dispersed, then gently rinsed with 10 mL of HBSS, the centrifugal tube was centrifuged to settle the cells, and finally the cells were resuspended in an experimental buffer.
   6) Cell density and viability were measured by Vi-cell.
   7) The GLP-1R/GIPR cells were diluted with the experimental buffer to a concentration of 2.0*10⁵/mL.
   8) 100 nL of the diluted cell suspension was transferred into the OptiPlate-384 plate.
   9) Incubation was performed at room temperature for 30 min.
d) An assay reagent was added:
   1) 10 µL of 800 nM gradient diluted cAMP standard was added into the empty wells of the OptiPlate-384 plate.
   2) 10 µL of cAMP assay reagent was added.
   3) The OptiPlate-384 plate was covered with TopSeal-Afilm and incubated at room temperature for 60 min.

The TopSeal-A film was removed, and readout was performed on EnVision.

### C. Experimental results

The experimental results were shown in Table 6 below.

**Table 6 In vitro GLP-1R/GIPR agonistic activity test results**

| **Peptide drugs** | **Agonistic activity on GLP-1R** | | | **Agonistic activity on GIPR** | | |
|---|---|---|---|---|---|---|
| | EC₅₀ (nM) | | Ratio | EC₅₀ (nM) | | Ratio |
| | (-) 1% HSA | (+) 1% HSA | | (-) 1% HSA | (+) 1% HSA | |
| **WX-001** | 0.05958 | 16.6 | 278.6 | 0.3239 | 15.97 | 49.3 |
| **WX-002** | 0.19 | 31.35 | 165 | 0.43 | 12.49 | 29 |
| **WX-003** | 0.54 | 13.97 | 26 | 1.07 | 43.95 | 41 |
| **WX-004** | 1.86 | 84.28 | 45 | 1.36 | 21.37 | 16 |
| **WX-005** | 0.036 | 7.63 | 212 | 0.16 | 19.29 | 121 |
| **WX-006** | 0.027 | NA | NA | 0.26 | NA | NA |
| **WX-007** | 0.026 | NA | NA | 0.40 | NA | NA |
| **WX-008** | 0.18 | NA | NA | 2.74 | NA | NA |
| **WX-009** | 0.014 | NA | NA | 0.074 | NA | NA |
| **WX-010** | 0.017 | NA | NA | 0.14 | NA | NA |
| **WX-011** | 0.018 | NA | NA | 0.25 | NA | NA |
| **WX-012** | 0.024 | NA | NA | 0.17 | NA | NA |
| **WX-013** | 0.057 | NA | NA | 0.18 | NA | NA |
| **WX-014** | 0.071 | NA | NA | 0.24 | NA | NA |

| | | | | | | |
|---|---|---|---|---|---|---|
| **Conclusion:** the compounds of the present application exhibit strong agonistic activity towards GLP-1R/GIPR. | | | | | | |

### Test example 2: Evaluation of pharmacokinetic profiles of the compounds in rats

### A. Experimental purpose:

To test the pharmacokinetic profiles of the compounds in SD rats.

### B. Experimental procedure

The pharmacokinetic characteristics in rodent animals following subcutaneous injection of the compounds were tested using standard protocols. In the experiment, the candidate compounds were formulated into clear solution, which was administered to rats by single subcutaneous injection (SC, 0.048 mpk). The vehicle for injection was citrate buffer solution (20 mM, pH=7). The whole blood was collected and processed to get the plasma. The drug concentration was analyzed by LC-MS/MS, and the pharmacokinetic parameters were calculated using Phoenix WinNonlin software.

### C. Experimental results

The experimental results were shown in Table 7 below.

**Table 7 Pharmacokinetic test results in rats**

| **Compound No.** | **Peak concentration of drugs (nM)** | **Half-life T_{1/2} (h)** | **Peak time Tmax (h)** | **SC concentration integration AUC₀₋₇₂ₕ (nM.hr)** |
|---|---|---|---|---|
| **WX005** | 28 | 21 | 16 | 1153 |
| **WX009** | 24 | 21 | 20 | 1092 |

| | | | | |
|---|---|---|---|---|
| Conclusion: the compounds of the present application exhibit excellent pharmacokinetic properties in rats. | | | | |

### Test example 3: Evaluation of pharmacokinetic profiles of the compounds in mice

### A. Experimental purpose:

To test the pharmacokinetic profiles of the compounds in C57BL/6 mice.

### B. Experimental procedure

The pharmacokinetic characteristics in rodent animals following subcutaneous injection of the compounds were tested using standard protocols. In the experiment, the candidate compounds were formulated into clear solution, which was administered by subcutaneous injection (SC, 0.048 mpk). The vehicle for subcutaneous injection was citrate buffer solution (20 mM, pH=7). The whole blood was collected and processed to get the plasma. The drug concentration was analyzed by LC-MS/MS, and the pharmacokinetic parameters were calculated using Phoenix WinNonlin software.

### C. Experimental results

The experimental results were shown in Table 8 below.

**Table 8 Pharmacokinetic test results in mice**

| **Compound No.** | **Peak concentration of drugs (nM)** | **Half-life T_{1/2} (h)** | **Peak time Tmax (h)** | **SC concentration integration AUC₀₋₇₂ₕ (nM.hr)** |
|---|---|---|---|---|
| **WX005** | 79 | 16 | 12 | 2240 |
| **WX009** | 59 | 17 | 12 | 1986 |

| | | | | |
|---|---|---|---|---|
| Conclusion: the compounds of the present application exhibit excellent pharmacokinetic properties in mice. | | | | |

### Test example 4: Evaluation of pharmacokinetic profiles of the compounds in Cynomolgus macaques

### A. Experimental purpose:

To test the pharmacokinetic profiles of the compounds in Cynomolgus macaques.

### B. Experimental procedure

The pharmacokinetic characteristics in mammals following intravenous injection and subcutaneous injection of the compounds were tested using standard protocols. In the experiment, the candidate compounds were formulated into clear solution, which was administered to Cynomolgus macaques by single subcutaneous injection (SC, 0.02 mpk). The vehicle for subcutaneous injection was citrate buffer solution (20 mM, pH=7). The whole blood was collected and processed to get the plasma. The drug concentration was analyzed by LC-MS/MS, and the pharmacokinetic parameters were calculated using Phoenix WinNonlin software.

### C. Experimental results

The experimental results were shown in Table 9 below.

**Table 9 Pharmacokinetic test results in Cynomolgus macaques**

| **Compound No.** | **Peak concentration of drugs (nM)** | **Half-life T_{1/2} (h)** | **Peak time Tmax (h)** | **SC concentration integration AUC₀₋₇₂ₕ (nM.hr)** |
|---|---|---|---|---|
| **WX005** | 36 | 100 | 24 | 4467 |
| **WX009** | 37 | 105 | 24 | 4737 |

| | | | | |
|---|---|---|---|---|
| Conclusion: the compounds of the present application exhibit excellent pharmacokinetic properties in monkeys. | | | | |

### Test example 5: Plasma stability test (PLS)

### A. Experimental purpose:

To study the stability of the test compounds in the plasma of normal mice.

### B. Experimental procedure

1. Before the experiment, the clotted frozen plasma was thawed in a water bath at 37°C. The resulting plasma was centrifuged at 4000 rpm for 5 min. If there is blood clot, remove the clot and adjust the pH to 7.4±0.1.
2. The test compound solution was prepared by diluting the compounds with DMSO to prepare a solution of 100 µM.
3. 98 µL of plasma, as a blank control, was added with 2 µL of the test compound solution (100 µM) to get a mixed solution with a final concentration of 2 µM, which was incubated in a water bath at 37°C.
4. 100 µL of H₃PO₄ solution and 800 µL of stop solution (a 100% solution of 200 ng/mL tolbutamide and 200 ng/mL labetalol in methanol) were respectively added at each time point (0, 10, 30, 60, and 120 min) to precipitate protein, which was then mixed thoroughly.
5. The samples were centrifuged at 4000 rpm for 20 min, and 100 µL of supernatant was taken from each well for LC-MS/MS analysis.

### C. Experimental results

The experimental results were shown in Table 10 below.

**Table 10 PLS test results**

| **Compound No.** | **WX005** | **WX009** |
|---|---|---|
| PLS (H/M) T_{1/2} (min) | >289.1/>289.1 | >289.1/>238.9 |

| | | |
|---|---|---|
| Conclusion: the compounds of the present application exhibit excellent plasma stability. | | |

### Test example 6: Plasma protein binding test (PPB)

### A. Experimental purpose:

To study the binding of the test compounds to human/mouse plasma albumin.

### B. Experimental procedure

1. Blank matrix preparation: On the day of the experiment, the plasma was thawed in cold water and centrifuged at 3220 rpm for 5 min to remove all the blood clots. The pH of the resulting plasma was measured and adjusted to 7.4±0.1 with 1% of phosphoric acid or 1 N of sodium hydroxide as desired.
2. Dilution of the test compounds: the test compounds were dissolved in dimethyl sulfoxide (DMSO) to prepare stock solutions of 10 mM and 2 mM, respectively. 2 µL of the stock solution (2 mM) was diluted with 98 µL of DMSO to obtain a working solution of 40 µM. 10 µL of the stock solution was diluted with 240 µL of DMSO to obtain a working solution of control compounds of 400 µM. A working solution of the compounds (5 µL) was mixed evenly with a blank matrix (995 µL) at a ratio of 1:200 to prepare a loading matrix.
3. Analytical procedure
   3.1 Equal amounts of 30 µL of the loading matrix (n=2) were transferred into a sample collection plate to prepare Time 0 (T0) samples for residue determination. The samples were immediately matched with corresponding blank buffer to a final volume of 60 µL, with a volume ratio of plasma (the loading matrix) to buffer being 1: 1 in each well. Then, into the T0 samples of the test compounds were respectively added 60 µL of 4% H₃PO₄ in H₂O and 480 µL of stop solution containing internal standard. They were then stored at 2°C to 8°C together with other samples for further treatment.
   3.2 The remaining plasma samples were pre-incubated in a carbon dioxide incubator at 37±1°C for 30 min. Protein-free samples (F samples) were prepared. Matrix-loaded samples (230 µL) were all transferred into polycarbonate tubes (n = 2) and ultracentrifuged at 37°C and 155,000 × g (35000 rpm) for 4 h.
   3.3 To prepare T samples (test samples), an additional matrix-containing sample was transferred into a separate 96-well plate (sample incubation plate) and incubated at 37°C for 4 h.
   3.4 At the end of centrifugation, 30 µL of protein-free samples (F samples) and 30 µL of T samples were taken from the second layer (beneath the upper layer) of the supernatant and transferred into a new sample collection plate. Each sample was mixed with corresponding blank buffer or matrix to a final volume of 60 µL, and the blank matrix : buffer volume ratio is 1:1. Into all the samples were added 60 µL of 4% H₃PO₄ solution in water and 480 µL of stop solution containing internal standard. The mixture was centrifuged at 4000 rpm for 20 min. 100 µL of supernatant for each sample was taken for LC-MS/MS analysis.

### C. Experimental results

The experimental results were shown in Table 11 below.

**Table 11 PPB test results**

| **Compound No.** | **WX005** | **WX009** |
|---|---|---|
| PPB% unbound (H/M) | NA/NA | NA/NA |

| | | |
|---|---|---|
| Note: NA indicates that the plasma protein binding was too high and no free drugs were detected at a normal plasma protein concentration. Conclusion: the compounds of the present application exhibit extremely high plasma protein binding. | | |

### Test example 7: Verification of efficacy in STZ-NASH mouse models

### A. Experimental purpose:

To verify the efficacy of the test compounds in STZ-HFD-induced NASH models of C57BL/6 mice.

### B. Experimental procedure

Modeling method: New-born mice were subcutaneously injected with STZ (200 µg per mouse) within 48 h of birth. After 4 weeks of breastfeeding, animals with fasting glucose values > 12 mmol/L were selected for 6 weeks of continuous HFD feeding to eventually establish the NASH model. Another 8 animals were selected without STZ injection and HFD feeding (normal control group).

Dosing regimen: After One week of HFD feeding, dosing began. The day of the first dosing was set as Day 1, followed by subcutaneous injections every two days for 5 consecutive weeks.

Experimental endpoint detection: pathological HE and SR staining.

### C. Experimental results

The experimental results were shown in Table 12 below.

**Table 12 Endpoint animal pathological indexes in STZ-NASH models**

| | **Model vehicle control groups** | **WX005** | **WX009** |
|---|---|---|---|
| Steatosis scores | 2 | 1.5 | 1.0 |
| Inflammation scores | 1.2 | 1.0 | 1.4 |
| Ballooning degeneration scores | 0.7 | 0.1 | 0 |
| NAS scores | 3.9 | 2.6 | 2.4 |
| Fibrosis (%) | 1.0 | 0.8 | 1.1 |

| | | | |
|---|---|---|---|
| Conclusion: the compounds of the present application are capable of significantly improving NAS scores in STZ-NASH mouse models. | | | |

## Claims

1. A polypeptide having a sequence as shown in formula (II),
YAibEGT FTSDY SIAibLD KIAQK AFVKW LIAGG PSSGA PPPS₀ **(II)**
which has modifications as below:
1) the amino acid at position i is replaced by lysine and then the amino group on the lysine at position i is attached to 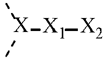 together with the amino group on the side chains of the lysine at position i+3, or the amino groups on the side chains of the lysine at positions j and j+4 are attached to 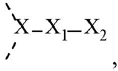 wherein i is 17, or wherein j is 20; and
2) additional 0 to 2 amino acids on the polypeptide of the sequence as shown in formula (II) are replaced; wherein,
Aib has a structure of 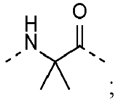
S₀ is selected from the group consisting of 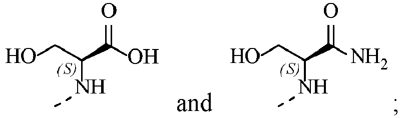
X is selected from the group consisting of 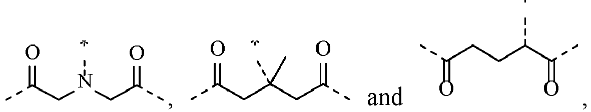
X₂ is selected from the group consisting of 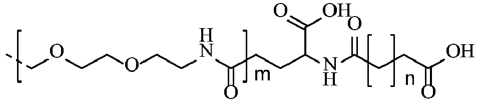 and 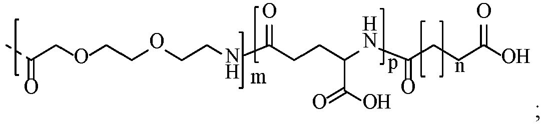 wherein, "*" indicates the position attached to X₁;
X₁ is selected from the group consisting of a single bond, -C(=O)-, -O-C(=O)-, and -N(R₁)-C(=O)-;
R₁ is selected from the group consisting of H and C₁₋₃ alkyl;
m is selected from 2, 3, and 4;
n is selected from 15, 16, 17, 18, and 19;
p is selected from 1 and 2.

2. The polypeptide according to claim 1, wherein the sequence of the polypeptide is as shown in formula (P),
YAibEGT FTSDY SIAibLD KKAQK AFVKW LIAGG PSSGA PPPS₀ **(P)**
which has modifications as below:
1) the amino groups on the side chains of the lysine at positions 17 and 20 are attached to 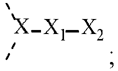 and
2) 0 to 2 amino acids on the polypeptide are replaced; wherein,
Aib has a structure of 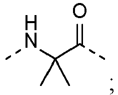
S₀ is selected from the group consisting of 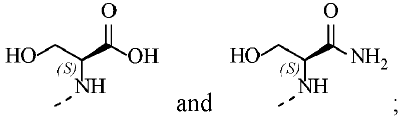
X is selected from the group consisting of 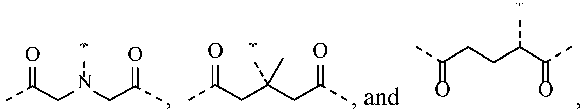 wherein, "*" indicates the position attached to X₁;
X₁ is selected from the group consisting of a single bond, -C(=O)-, -O-C(=O)-, and -N(R₁)-C(=O)-;
R₁ is selected from the group consisting of H and C₁₋₃ alkyl;
X₂ is selected from the group consisting of 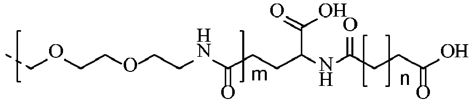 and 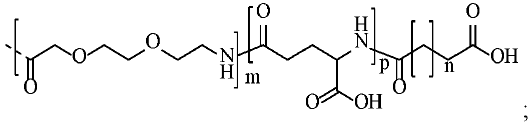
m is selected from 2, 3, and 4;
n is selected from 15, 16, 17, 18, and 19;
p is selected from 1 and 2.

3. The polypeptide according to claim 1 or 2, wherein, 0 to 2 amino acids at position 21, 23 or 24 of the polypeptide are replaced.

4. The polypeptide according to claim 1, wherein the polypeptide has a sequence selected from the group consisting of sequences as shown in formulas (II-1), (II-2), (II-3), (II-4), (III-6), (IV-7), (IV-8), (IV-9), and (IV-10),
YAibEGT FTSDY SIAibLD KIAQK AFVKW LIAGG PSSGA PPPS-NH₂ **(II-1)**
YAibEGT FTSDY SIAibLD KIAQK EFVKW LIAGG PSSGA PPPS-NH₂ **(II-2)**
YAibEGT FTSDY SIAibLD KIAQK AFIKW LIAGG PSSGA PPPS-NH₂ **(II-3)**
YAibEGT FTSDY SIAibLD KIAQK AFVKW LLAGG PSSGA PPPS-NH₂ **(II-4)**
YAibEGT FTSDY SIAibLD KKAQK AFVEW LIAGG PSSGA PPPS-NH₂ **(III-6)**
YAibEGT FTSDY SIAibLD KKAQK AFVQW LIAGG PSSGA PPPS-NH₂ **(IV-7)**
YAibEGT FTSDY SIAibLD KKAQK AFVAW LIAGG PSSGA PPPS-NH₂ **(IV-8)**
YAibEGT FTSDY SIAibLD KKAQK AFVIW LIAGG PSSGA PPPS-NH₂ **(IV-9)**
YAibEGT FTSDY SIAibLD KKAQK EFVEW LIAGG PSSGA PPPS-NH₂ **(IV-10)**
which have modifications as below:
the amino groups on the side chains of the lysine at positions 17 and 20 or the amino groups on the side chains of the lysine at positions 20 and 24 are attached to 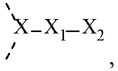 wherein,
Aib, X, X₁, and X₂ are as defined in claim 1.

5. The polypeptide according to any one of claims 1 to 4, wherein, R₁ is selected from H.

6. The polypeptide according to any one of claims 1 to 4, wherein, X₁ is selected from the group consisting of a single bond, -C(=O)-, -O-C(=O)-, and -NH-C(=O)-.

7. The polypeptide according to any one of claims 1 to 4, wherein, m is selected from 2.

8. The polypeptide according to any one of claims 1 to 4, wherein, n is selected from 15 and 17.

9. The polypeptide according to any one of claims 1 to 4, wherein, p is selected from 2.

10. The polypeptide according to any one of claims 1 to 4, wherein, X₂ is selected from the group consisting of 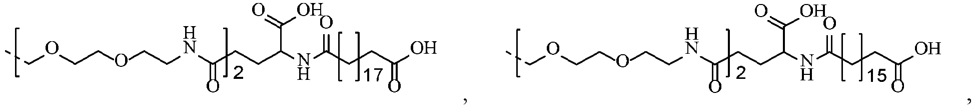 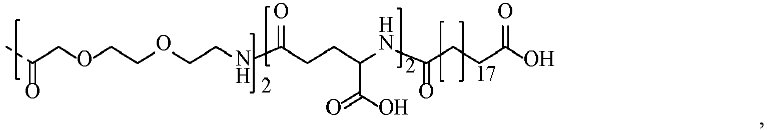 and

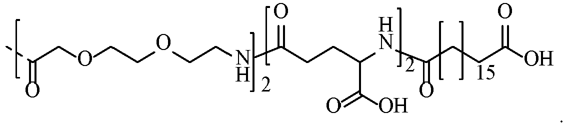

11. The polypeptide according to claim 10, wherein, X₂ is selected from the group consisting of 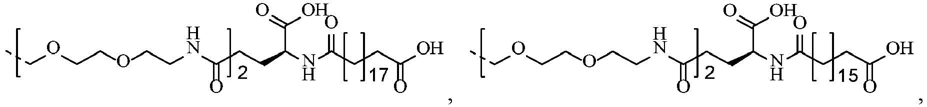 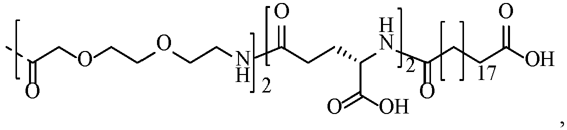 and

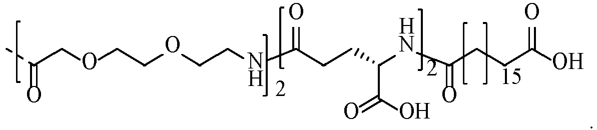

12. The polypeptide according to any one of claims 1 to 4, wherein, the amino groups on the side chains of the lysine at positions i and i+3 or the amino groups on the side chains of the lysine at positions j and j+4 are attached to 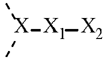 to form

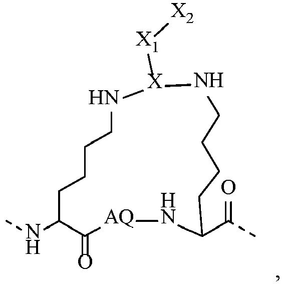

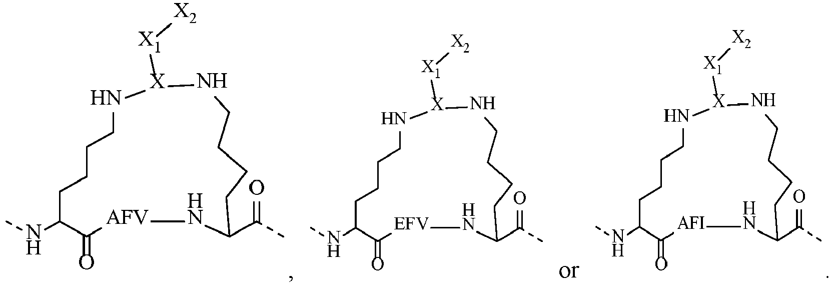

13. The polypeptide according to any one of claims 1 to 4, wherein, the amino groups on the side chains of the lysine at positions i and i+3 or the amino groups on the side chains of the lysine at positions j and j+4 are attached to 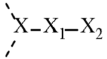 to form

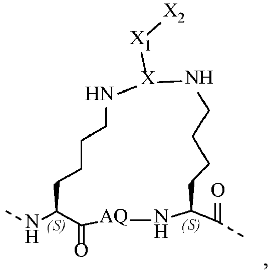

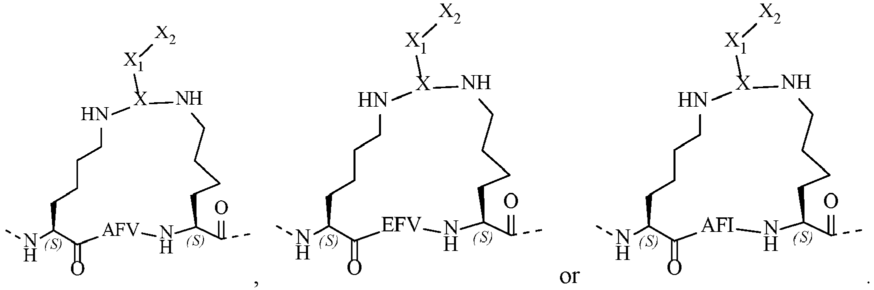

14. The polypeptide according to any one of claims 1 to 4, wherein, the structural unit 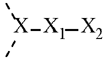 is selected from the group consisting of 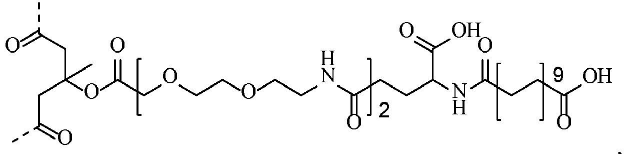 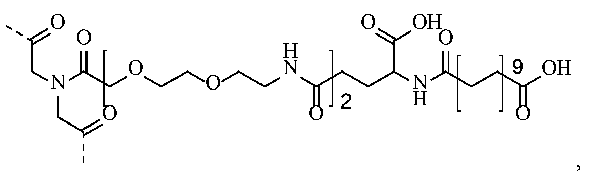 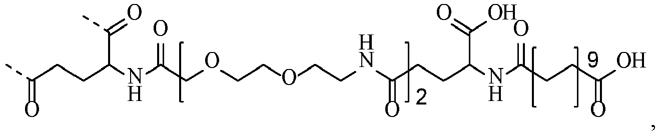 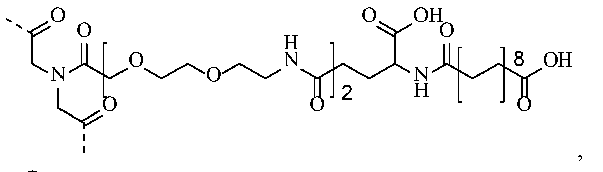 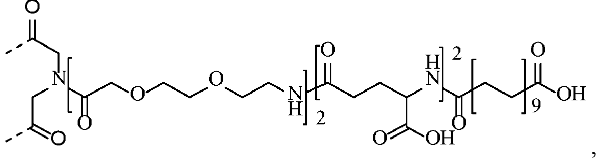 and

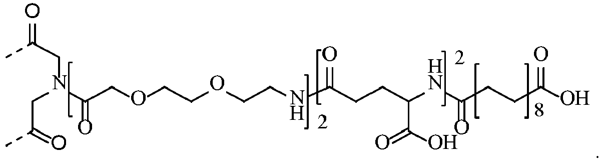

15. The polypeptide according to claim 14, wherein, the structural unit 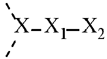 is selected from the group consisting of 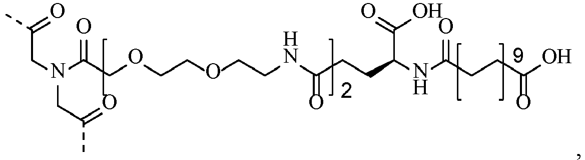 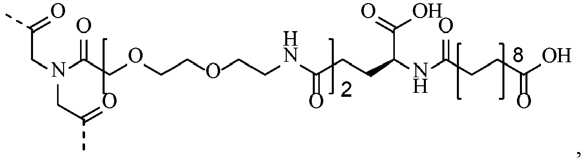 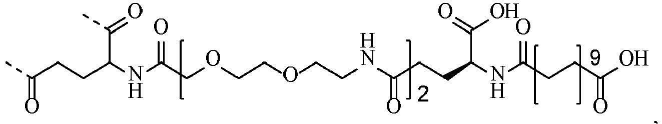 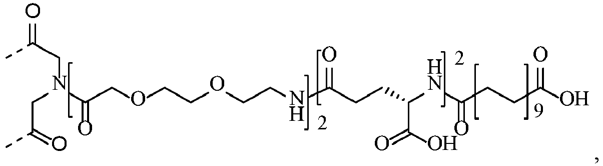 and

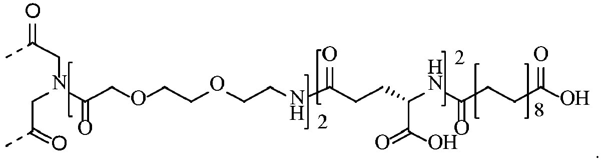

16. A Polypeptide having a formula selected from the group consisting of formulas as shown below, 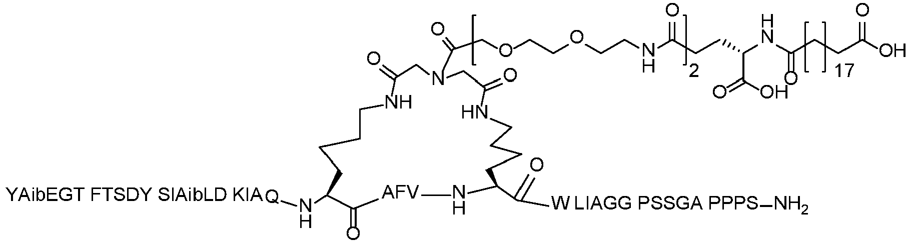 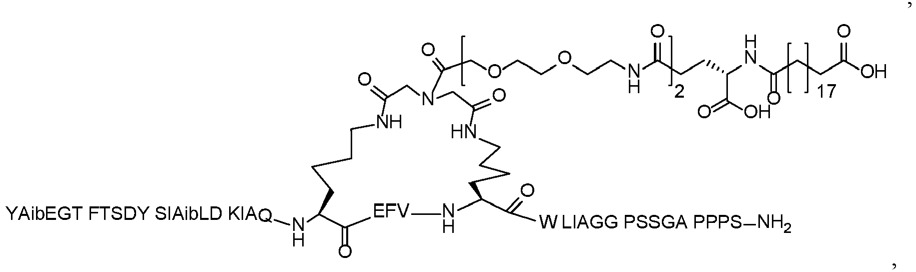 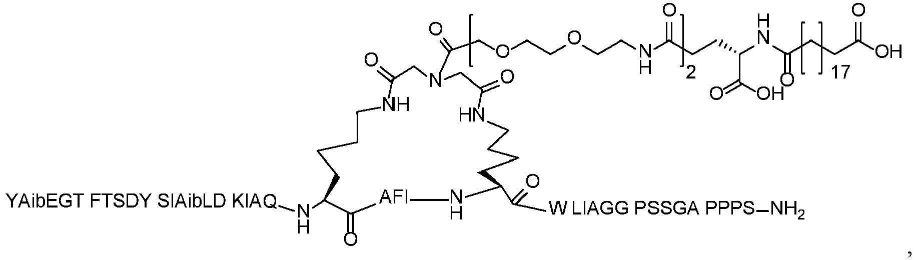 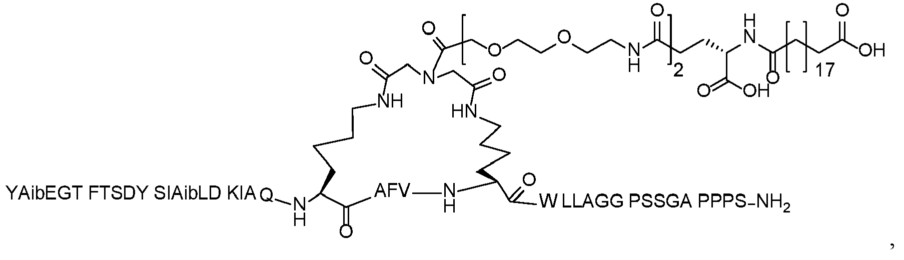 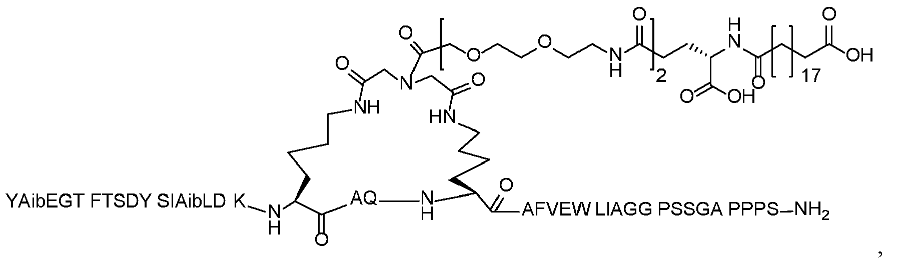 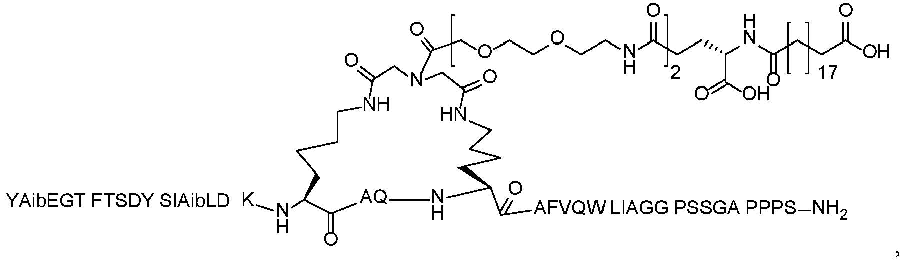 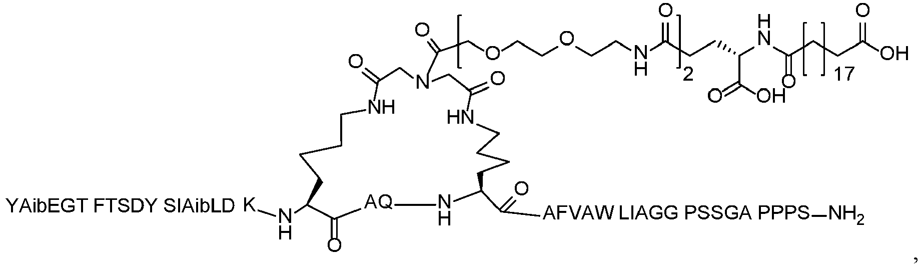 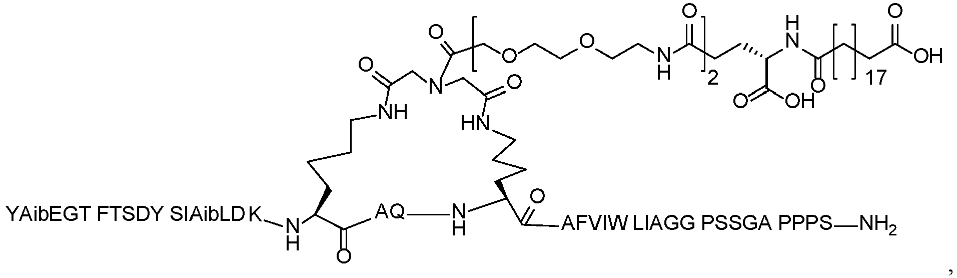 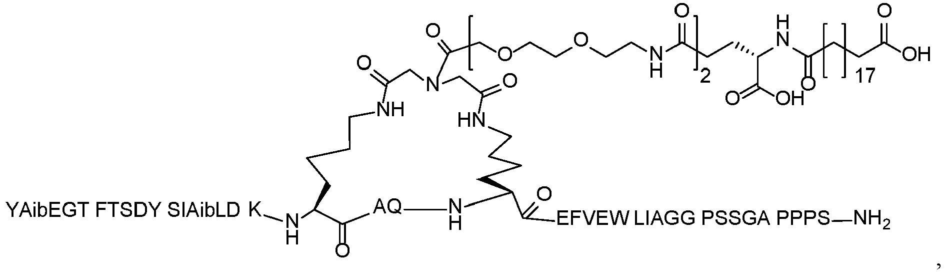 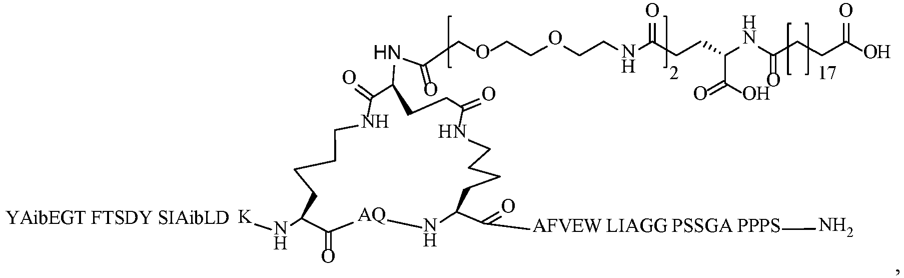 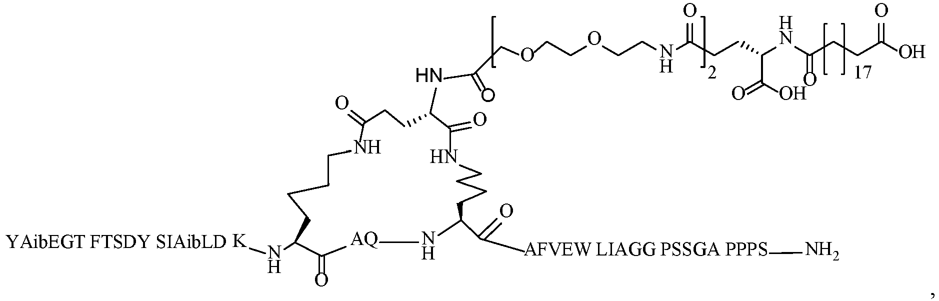 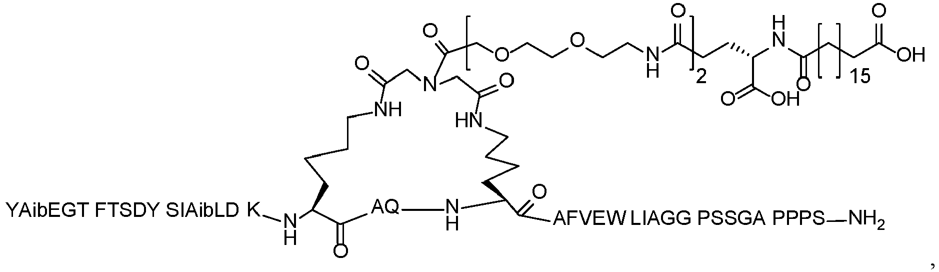 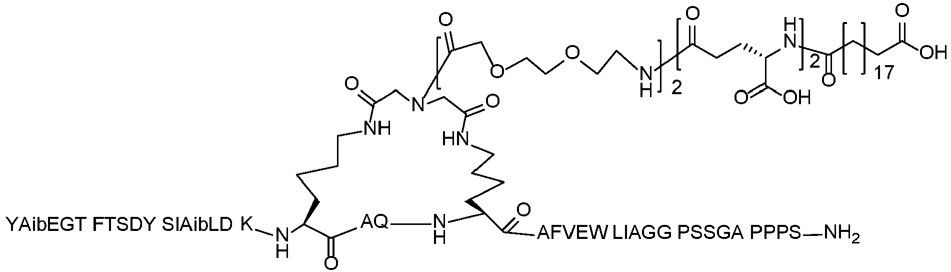 and

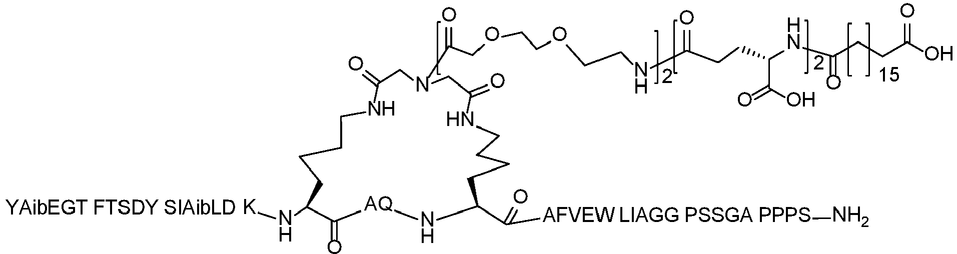

17. A pharmaceutical composition, comprising a therapeutically effective amount of the polypeptide of any one of claims 1 to 16 or a pharmaceutically acceptable salt thereof, as an active ingredient, and a pharmaceutically acceptable carrier.

18. Application of the polypeptide of any one of claims 1 to 16 or a pharmaceutically acceptable salt thereof or the pharmaceutical composition of claim 17 in the preparation of drugs for treating NAFLD and NASH.

19. The polypeptide of any one of claims 1 to 16 or a pharmaceutically acceptable salt thereof or the pharmaceutical composition of claim 17, being administered once every 3 days, once every 4 days, once a week, or once every two weeks.
